# EUROPEAN PATENT SPECIFICATION

(11) **EP 3 537 895 B1**
(45) Date of publication and mention of the grant of the patent: **06.03.2024**
(21) Application number: 17870309.6
(22) Date of filing: 08.11.2017
(51) Int. Cl.: C07J 17/00, A23L 2/60, B01D 15/16, B01D 15/32

(54) **NOVEL MOGROSIDES AND USE THEREOF**
NEUARTIGE MOGROSIDE UND VERWENDUNG DAVON
NOUVEAUX MOGROSIDES ET LEUR UTILISATION

(30) Priority: 08.11.2016 US 201662419238 P
(43) Date of publication of application: 18.09.2019
(73) Proprietor: PureCircle USA Inc., Westchester, IL 60154 (US)
(72) Inventor: MARKOSYAN, Avetik, 0014 Yerevan (AM); CHOW, Siew, Yin, 41050 Klang, Selangor (MY); RAMANDACH, Saravanan, 70400 Seremban, Negeri Sembilan (MY)
(74) Representative: Novagraaf Group
(86) International application number: PCT/US2017/060604
(87) International publication number: WO 2018/089469

(56) References cited:
- WO-A1-2016/038617
- WO-A2-2014/146089
- WO-A2-2017/075257
- CN-A- 104 926 907
- US-A1- 2014 170 286
- US-A1- 2016 029 678
- US-B2- 9 101 162
- VENKATA SAI PRAKASH CHATURVEDULA ET AL: "Cucurbitane Glycosides from Siraitia grosvenorii", JOURNAL OF CARBOHYDRATE CHEMISTRY, vol. 30, no. 1, 1 January 2011 (2011-01-01), pages 16-26, XP55292751, UK ISSN: 0732-8303, DOI: 10.1080/07328303.2011.583511
- HONGYANG ZHANG ET AL: "Identification of flavonol and triterpene glycosides in Luo-Han-Guo extract using ultra-high performance liquid chromatography/quadrupole time-of-flight mass spectrometry", JOURNAL OF FOOD COMPOSITION AND ANALYSIS, vol. 25, no. 2, 1 March 2012 (2012-03-01), pages 142-148, XP55681112, AMSTERDAM, NL ISSN: 0889-1575, DOI: 10.1016/j.jfca.2011.09.004
- FU LI ET AL: "Cucurbitane glycosides from the fruit of Siraitia grosvenori and their effects on glucose uptake in human HepG2 cells in vitro", FOOD CHEMISTRY, vol. 228, 10 February 2017 (2017-02-10), pages 567-573, XP55681030, NL ISSN: 0308-8146, DOI: 10.1016/j.foodchem.2017.02.018

## Description

### Field of the Invention

The invention relates to a process for purification of novel mogrosides from *Siraitia grosvenorii* fruit extract, and their applications in foods, beverage and other consumables.

### Description of the Related Art

Luo han guo generally refers to a fruit of *Siraitia grosvenorii,* a member of the *Cucurbitaceae* family, which is a plant native to some regions of southern Asia and China. The sweet taste of luo han guo mainly comes from triterpene glycosides generally known as mogrosides or mogrol glycosides. Mogrosides comprise only about 1% of the luo han guo fruit. There are a number of mogrosides identified in luo han guo but generally mogroside V (CAS No: 88901-36-4) has the highest concentration compared to others (Table 1). Mogrol glycosides have the same core molecule - mogrol or oxo-mogrol and differ from each other by number and type of residues bonded to mogrol or oxo-mogrol molecules.

In particular, J. Food Compn. Anal. Vol 25(2) pp 142-148 (2012), J. Carbohydr. Chem. Vol 30(1) pp 16-26 (2011) and WO-A-2016/038617 all disclose Mogroside compounds and their sweet taste.

**Table 1**

| Mogrosides present in Luo han guo fruits | | |
|---|---|---|
| **Substance** | **Mol. Formula** | **Mol. Weight** |
| Mogroside IIE | C₄₂H₇₂O₁₄ | 801.01 |
| Mogroside III | C₄₈H₈₂O₁₉ | 963.15 |
| Mogroside IV | C₅₄H₉₂O₂₄ | 1125.29 |
| Mogroside V | C₆₀H₁₀₂O₂₉ | 1287.43 |
| Mogroside VI | C₆₆H₁₁₂O₃₄ | 1449.58 |
| 11-oxo-Mogroside V | C₆₀H₁₀₀O₂₉ | 1285.42 |
| Siamenoside I | C₅₄H₉₂O₂₄ | 1125.29 |
| Grosmomoside I | C₅₄H₉₂O₂₄ | 1125.29 |

Various extraction techniques are used to isolate mogrosides from luo han guo fruits. As a result luo han guo powdered extracts are being prepared which usually contain 30-65% w/w of total mogrosides, and mogroside V content of those materials can vary as much as 18-55%. Such extracts generally cannot be widely used in foods and beverages as they possess undesirable organoleptic properties.

Hence there is a need for compositions comprising novel mogroside molecules with significantly improved organoleptic characteristics allowing their wider usage in foods, beverages and other consumables.

No techniques are currently available for purifying novel mogrosides. Therefore, there is a need for a process of for purification of novel high purity mogrosides from *Siraitia grosvenori* fruit extract.

### Brief description of the drawings

Of the compounds referred to below, compounds (8), (9), (11), (12), (14) and (15) are compounds of the present invention, whereas all other compounds referred to are for the purposes of comparison only.

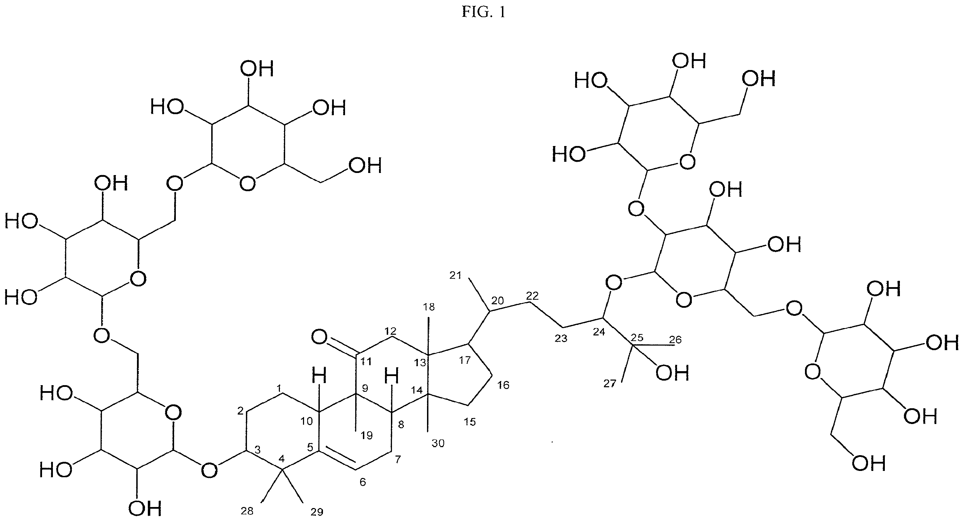

FIG. 1: Chemical structure of novel mogroside compound 1

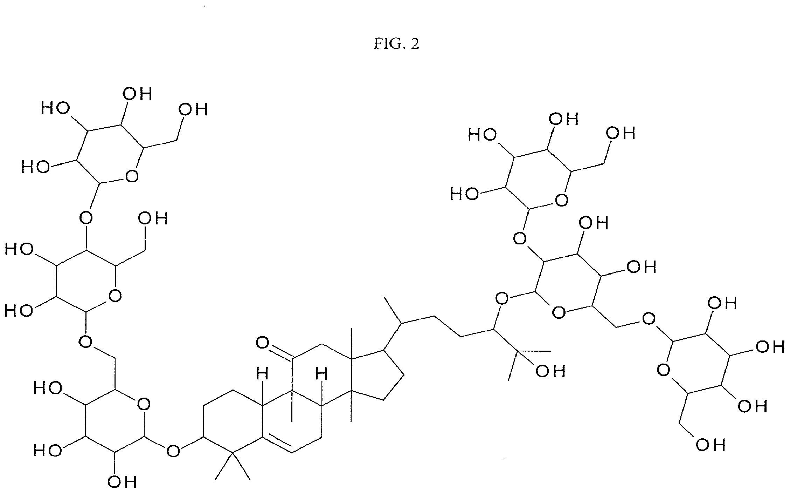

FIG. 2: Chemical structure of novel mogroside compound 2

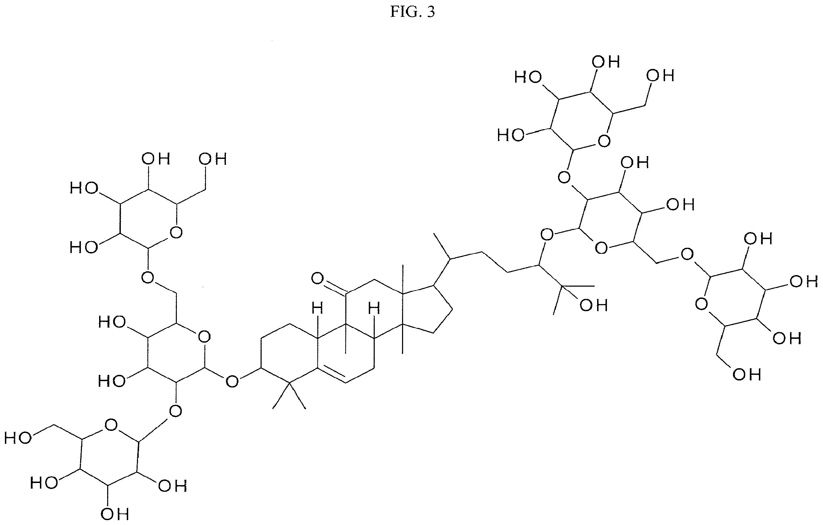

FIG. 3: Chemical structure of novel mogroside compound 3

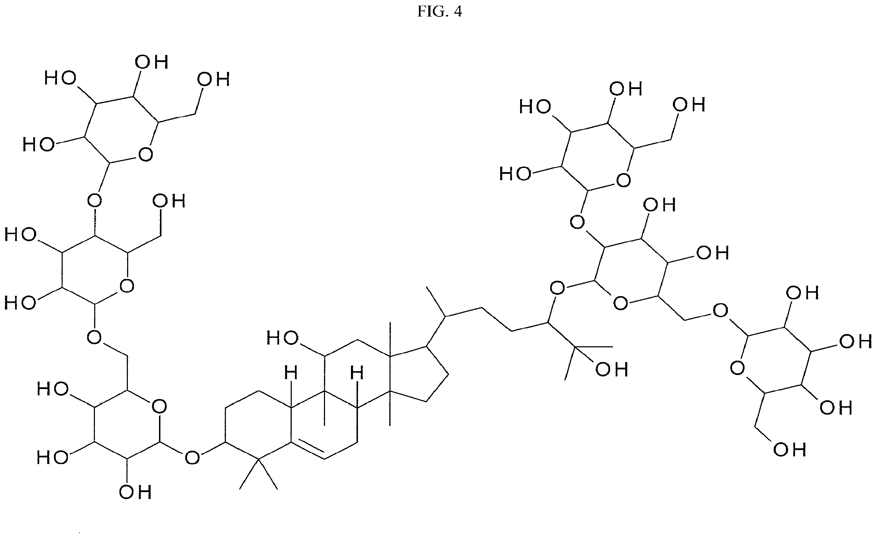

FIG. 4: Chemical structure of novel mogroside compound 4

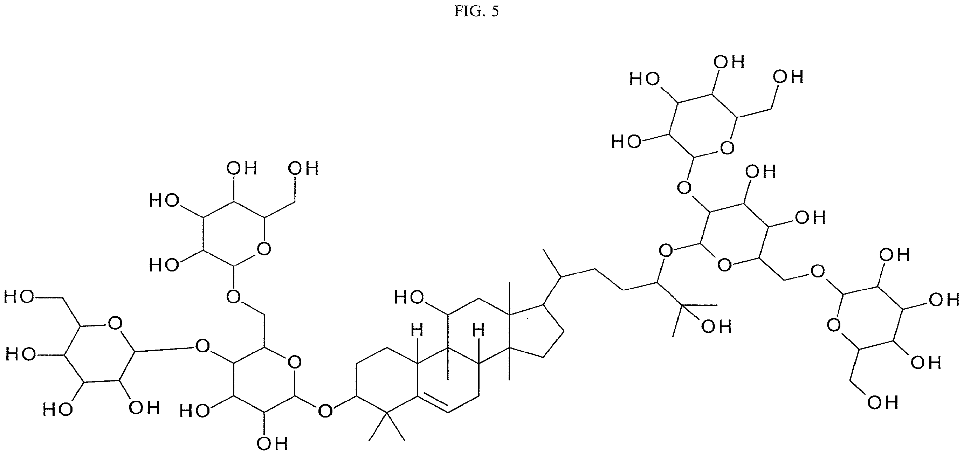

FIG. 5: Chemical structure of novel mogroside compound 5

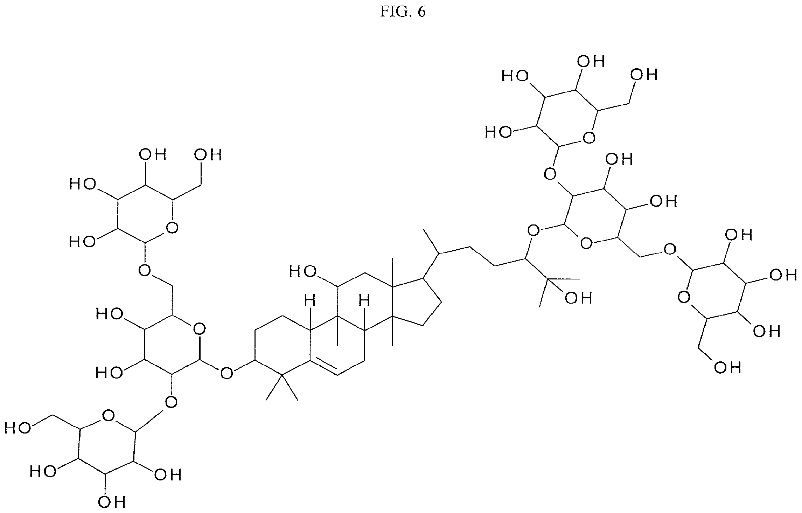

FIG. 6: Chemical structure of novel mogroside compound 6

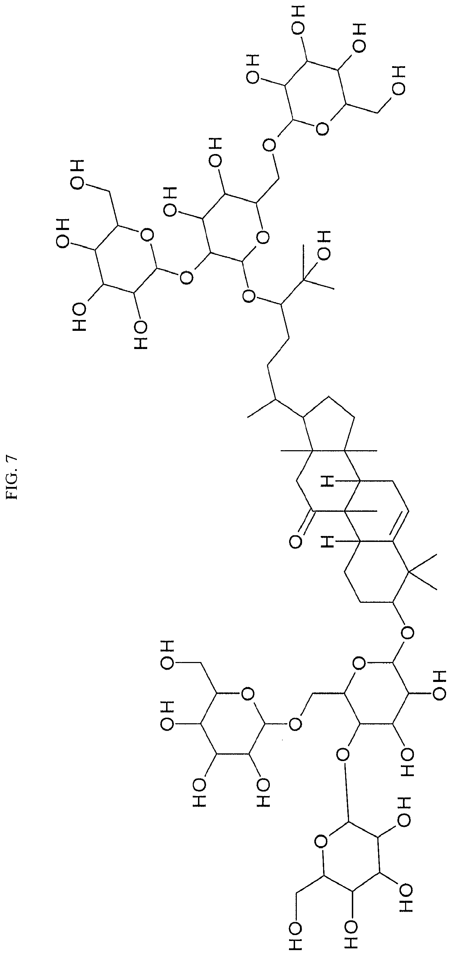

FIG. 7: Chemical structure of novel mogroside compound 7
FIG. 8: Chemical structure of novel mogroside compound 8
FIG. 9: Chemical structure of novel mogroside compound 9

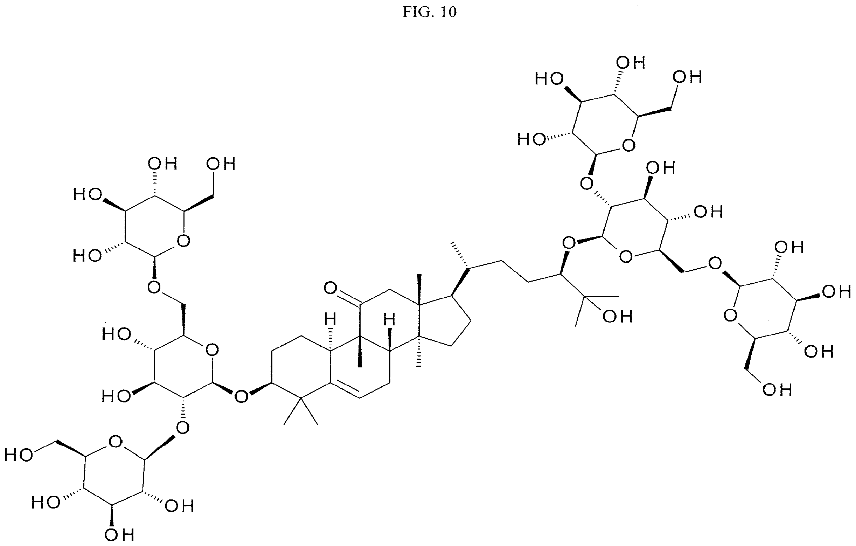

FIG. 10: Chemical structure of novel mogroside compound 10
FIG. 11: Chemical structure of novel mogroside compound 11
FIG. 12: Chemical structure of novel mogroside compound 12

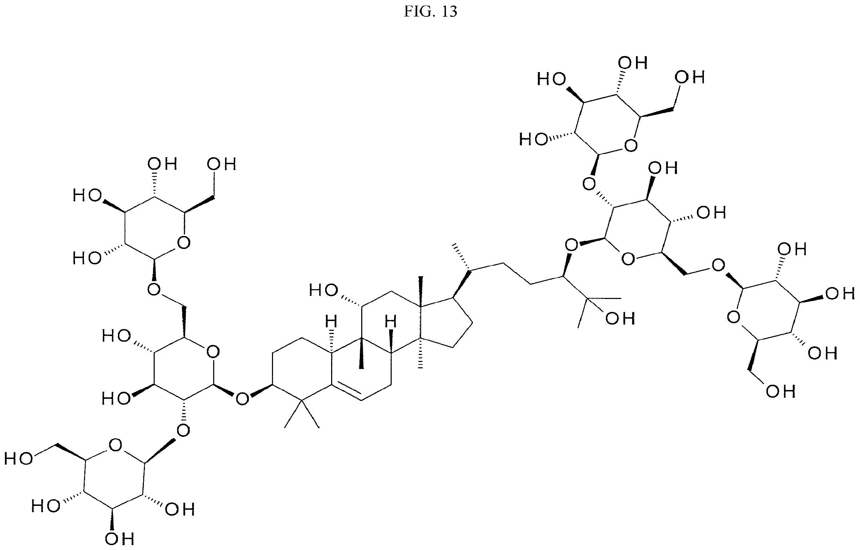

FIG. 13: Chemical structure of novel mogroside compound 13
FIG. 14: Chemical structure of novel mogroside compound 14
FIG. 15: Chemical structure of novel mogroside compound 15

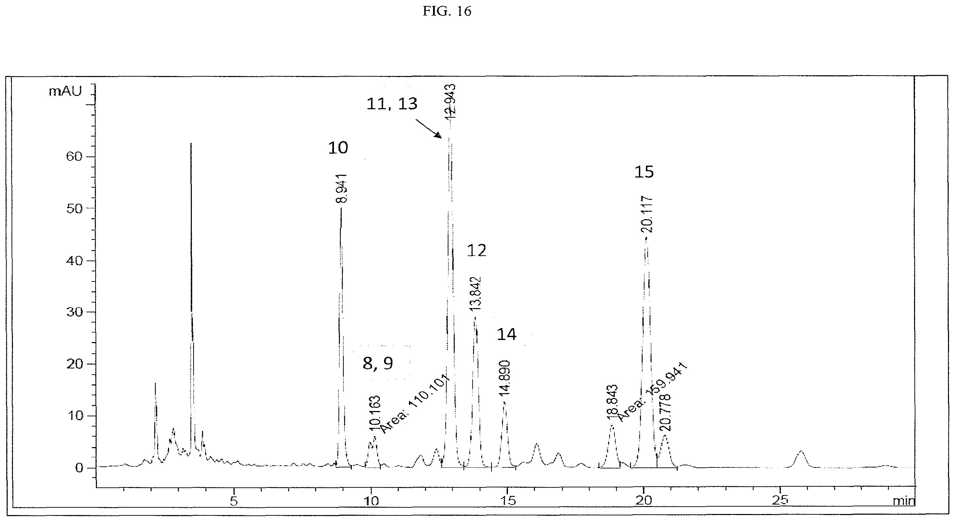

FIG. 16: Analytical-Preparative HPLC of mogroside mixtures

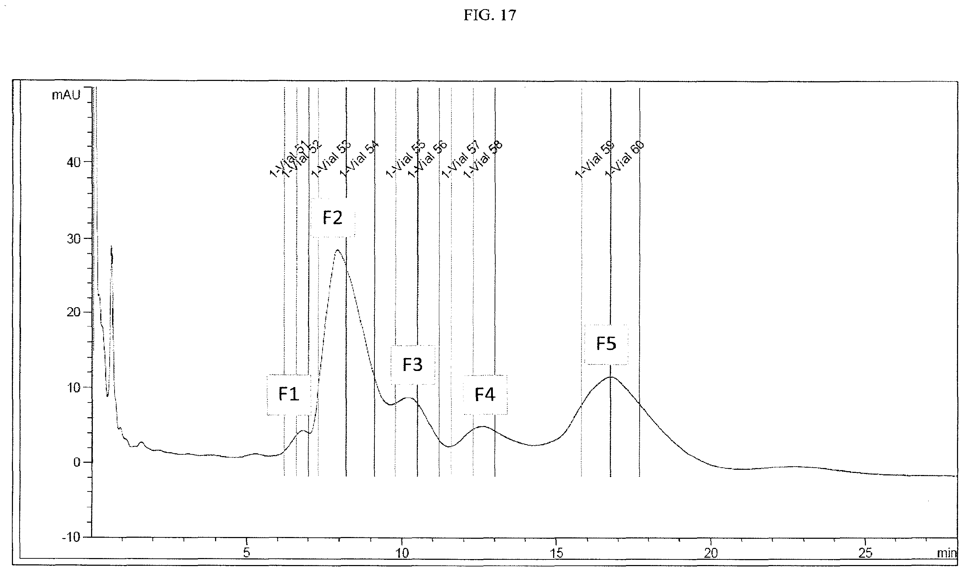

FIG. 17:Preparative HPLC of mogroside mixtures

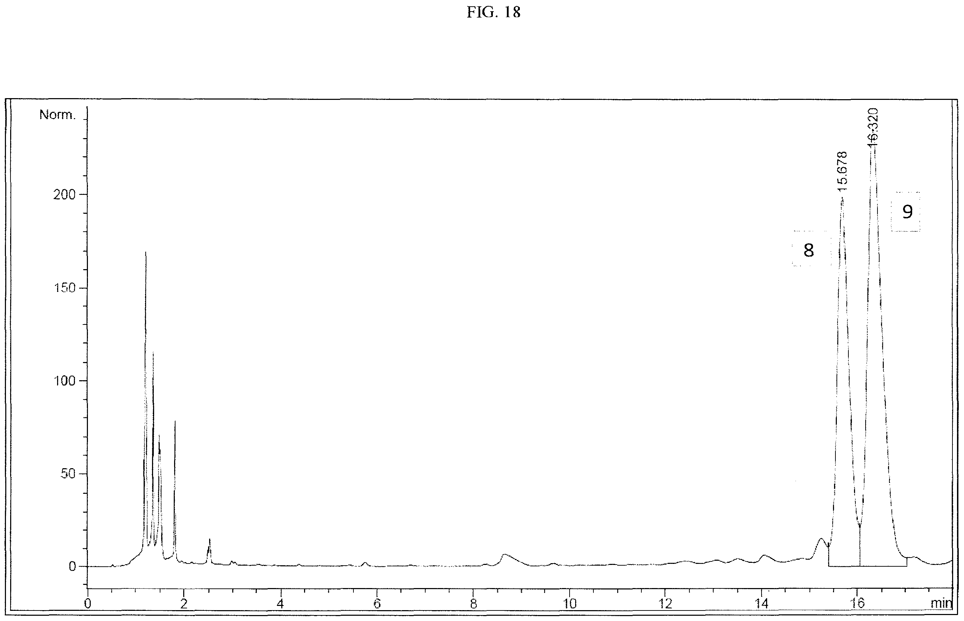

FIG. 18: Analytical-Preparative HPLC of Fraction 1 containing compounds 8 and 9

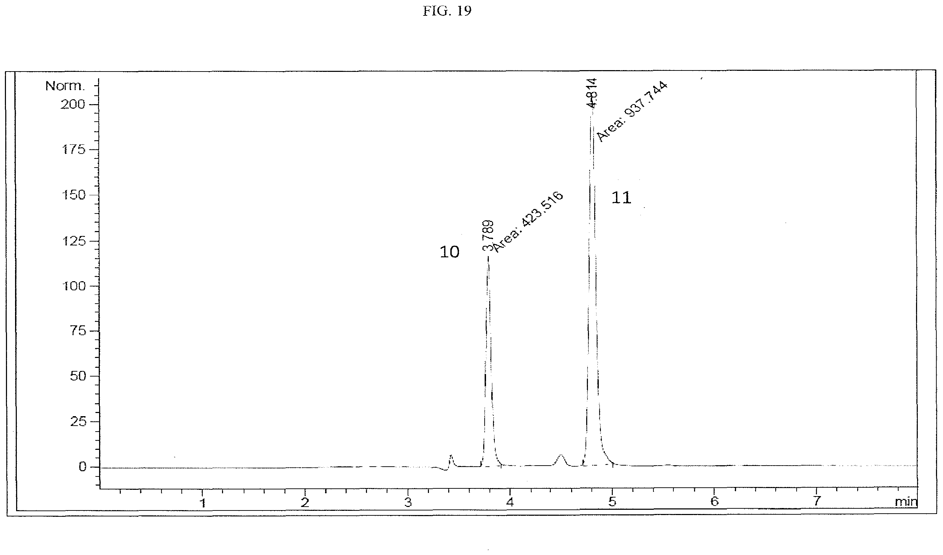

FIG 19: Analytical-Preparative HPLC of Fraction 2 containing compounds 10 and 11

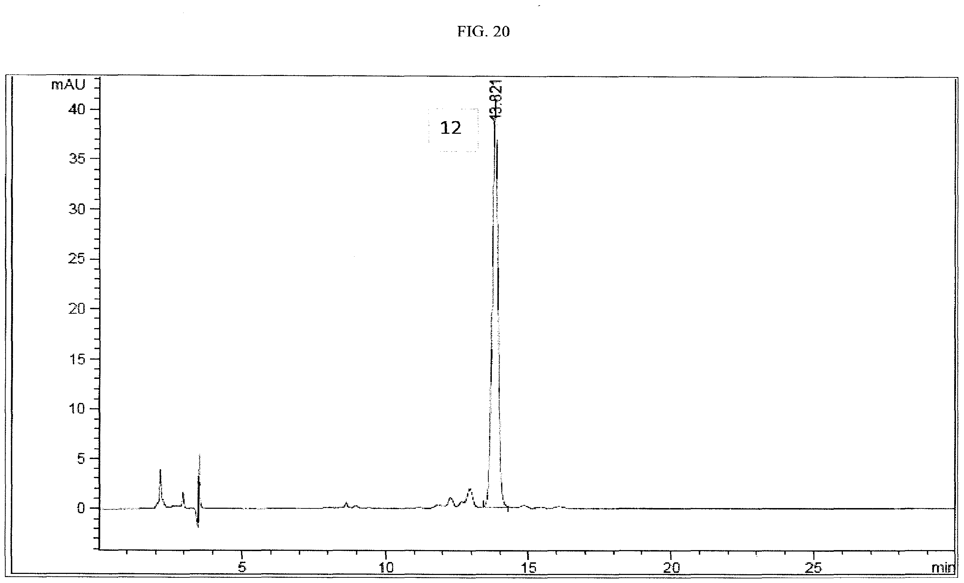

FIG 20: Analytical-Preparative HPLC of Fraction 3 containing compound 12

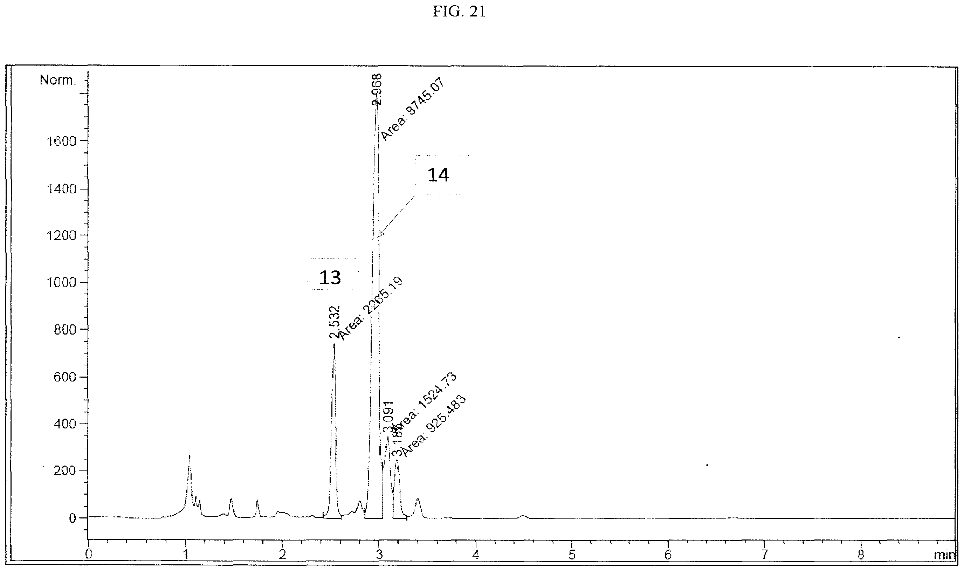

FIG 21: Analytical-Preparative HPLC of Fraction 4 containing compounds 13, 14

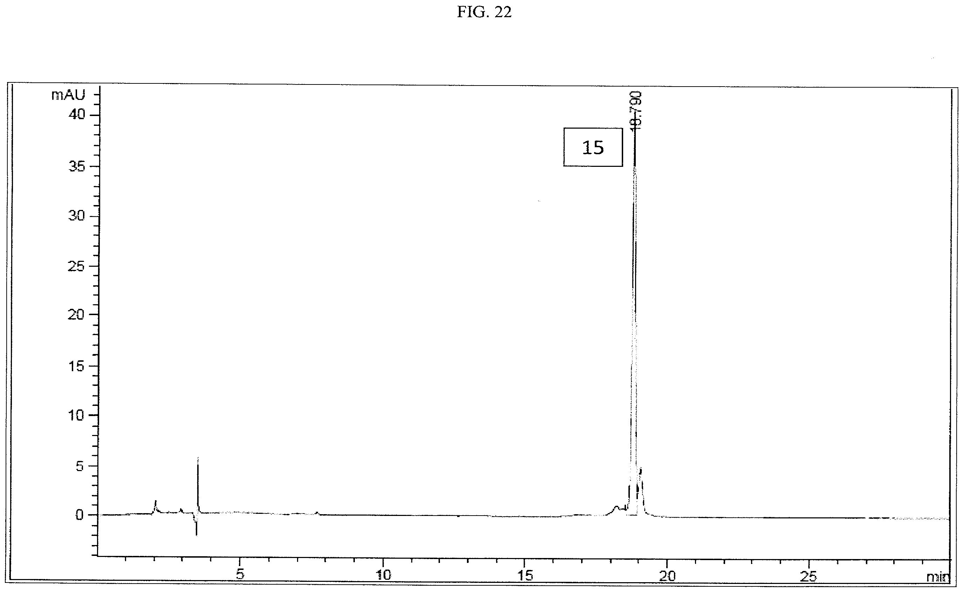

FIG 22: Analytical-Preparative HPLC of Fraction 5 containing compound 15.

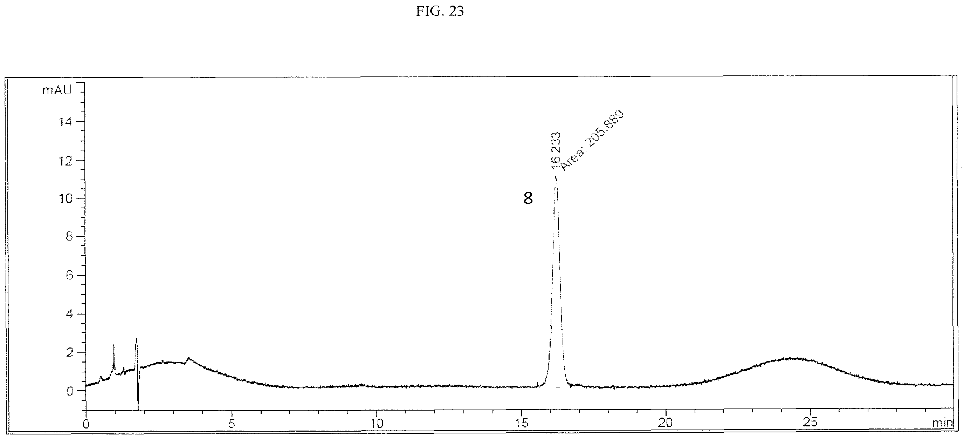

FIG. 23: Analytical-Preparative HPLC of compound 8

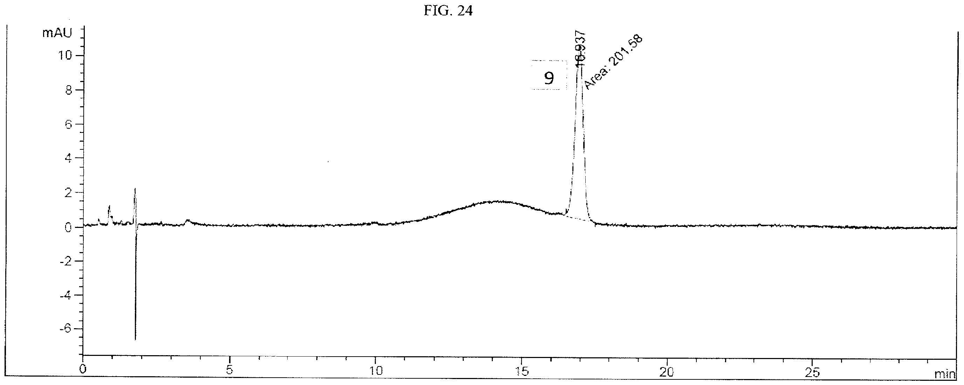

FIG. 24: Analytical-Preparative HPLC of compound 9

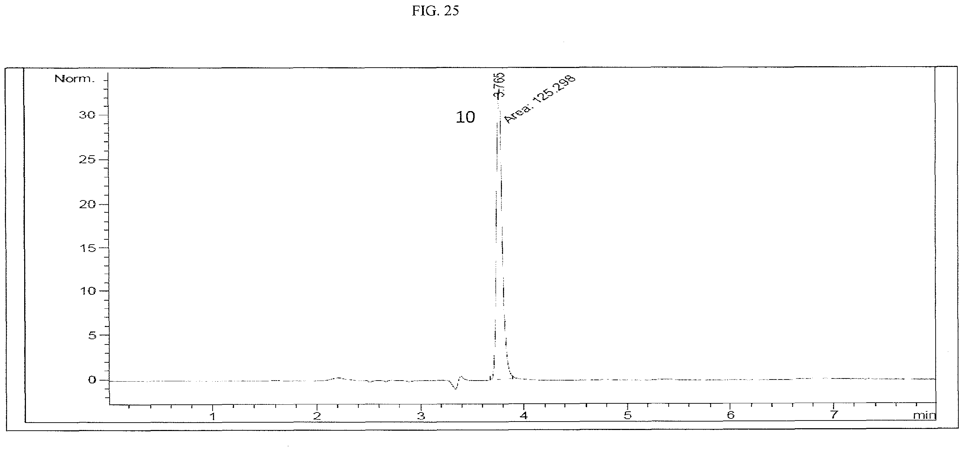

FIG. 25: Analytical-Preparative HPLC of compound 10

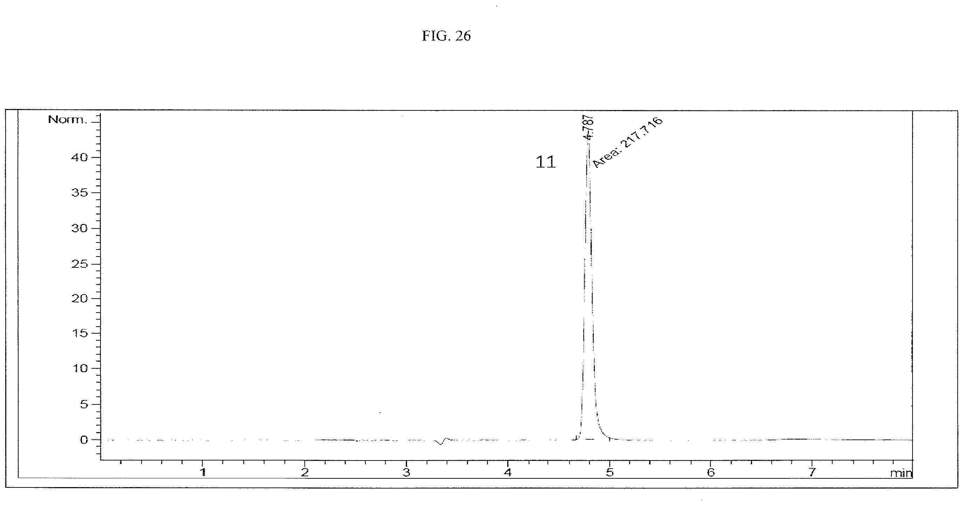

FIG. 26: Analytical-Preparative HPLC of compound 11

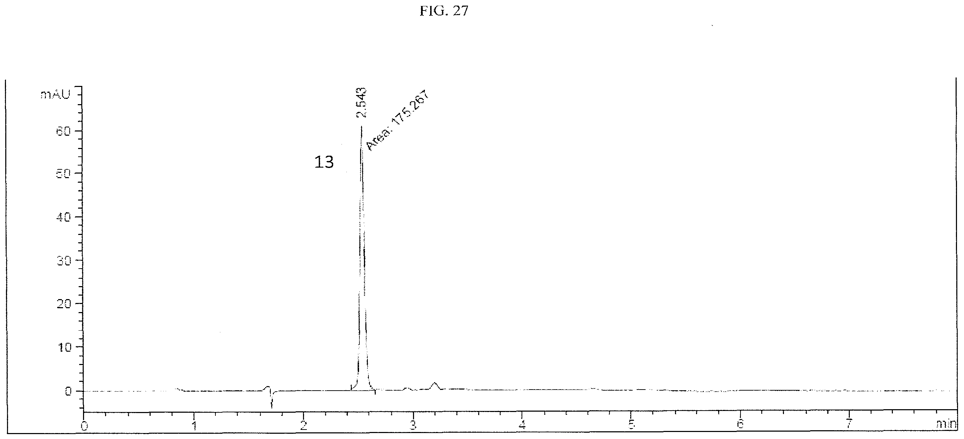

FIG. 27: Analytical-Preparative HPLC of compound 13

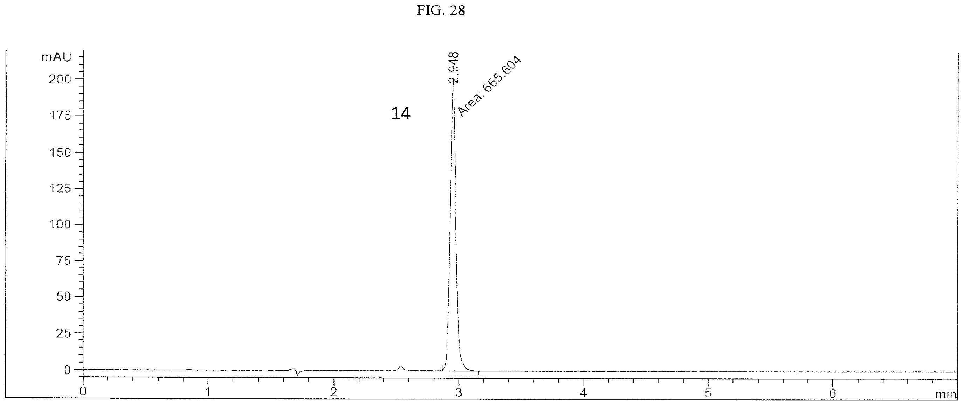

FIG. 28: Analytical-Preparative HPLC of compound 14

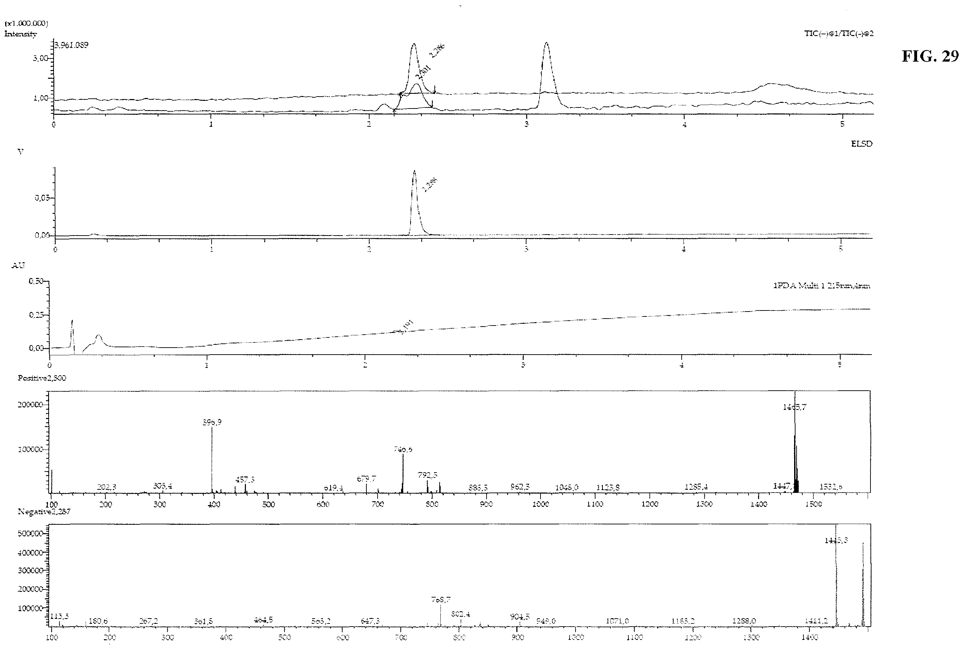

FIG. 29: LCMS of compound 8

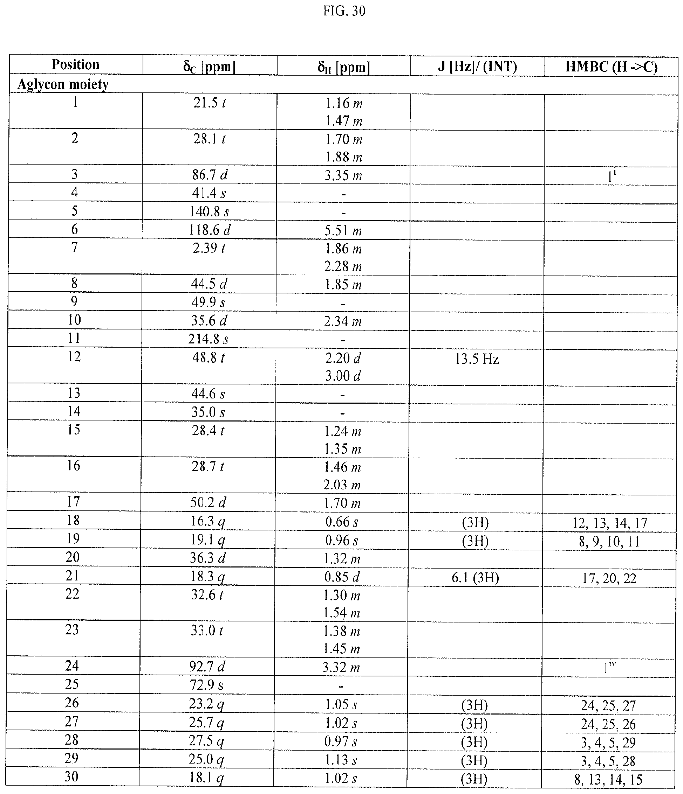

FIG. 30: NMR data of compound 8

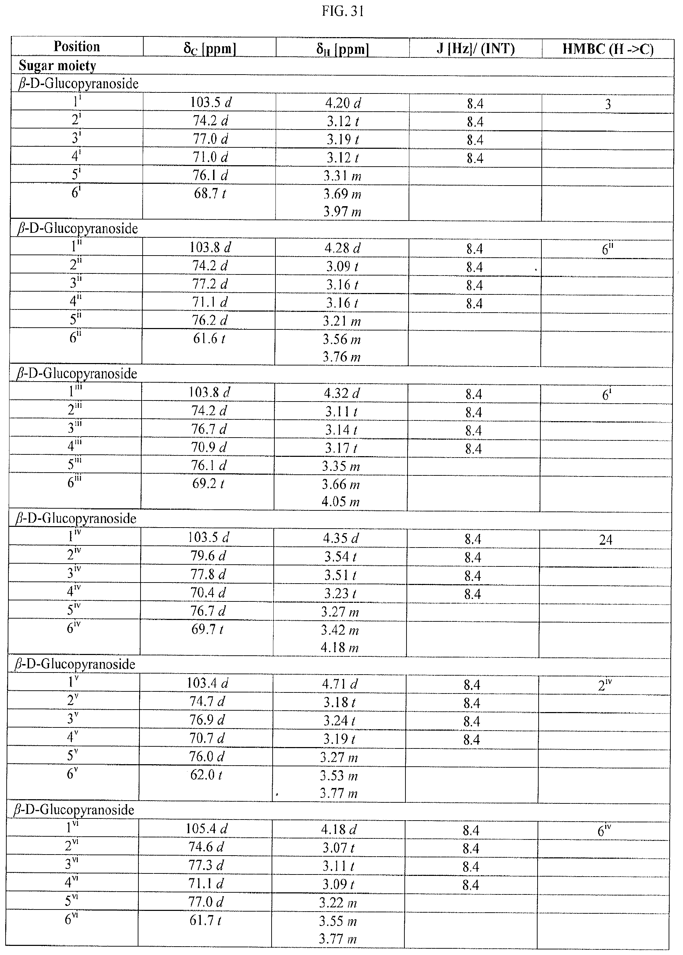

FIG. 31: NMR data of compound 8 (continued)

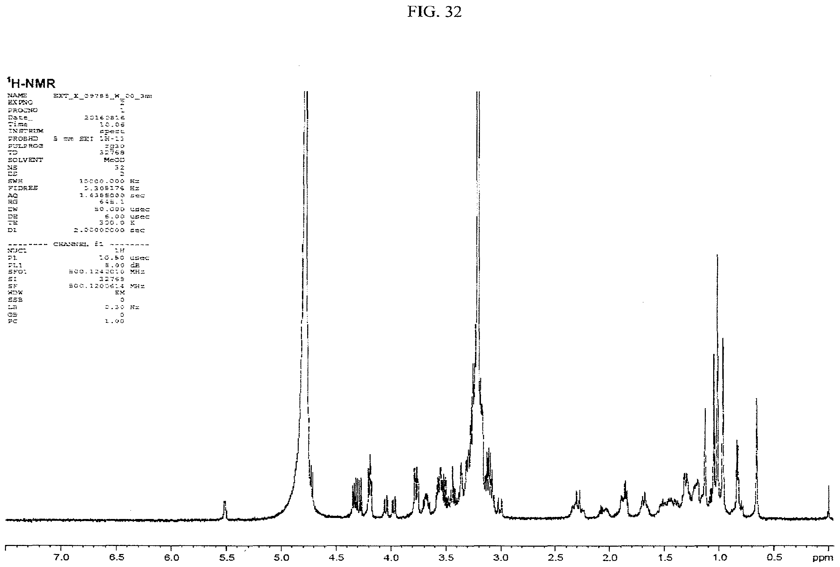

FIG. 32: 1H NMR spectrum of compound 8

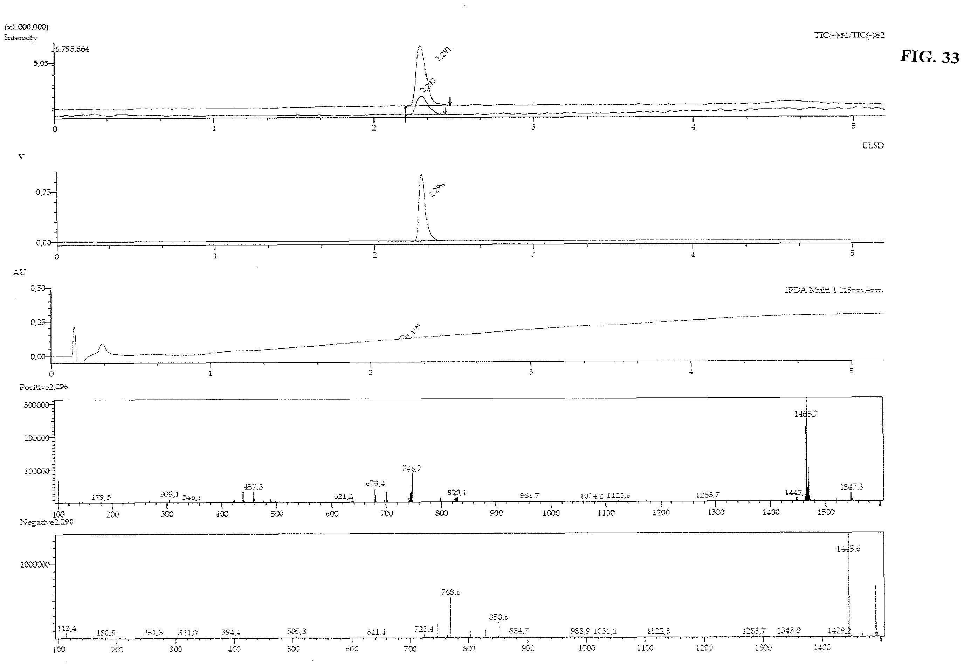

FIG. 33: LCMS of compound 9

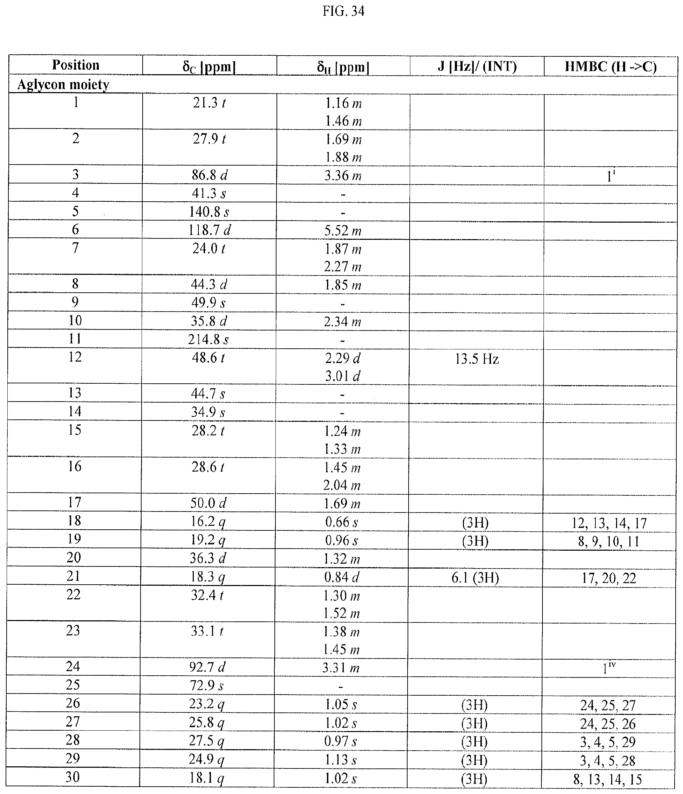

FIG. 34: NMR data of compound 9

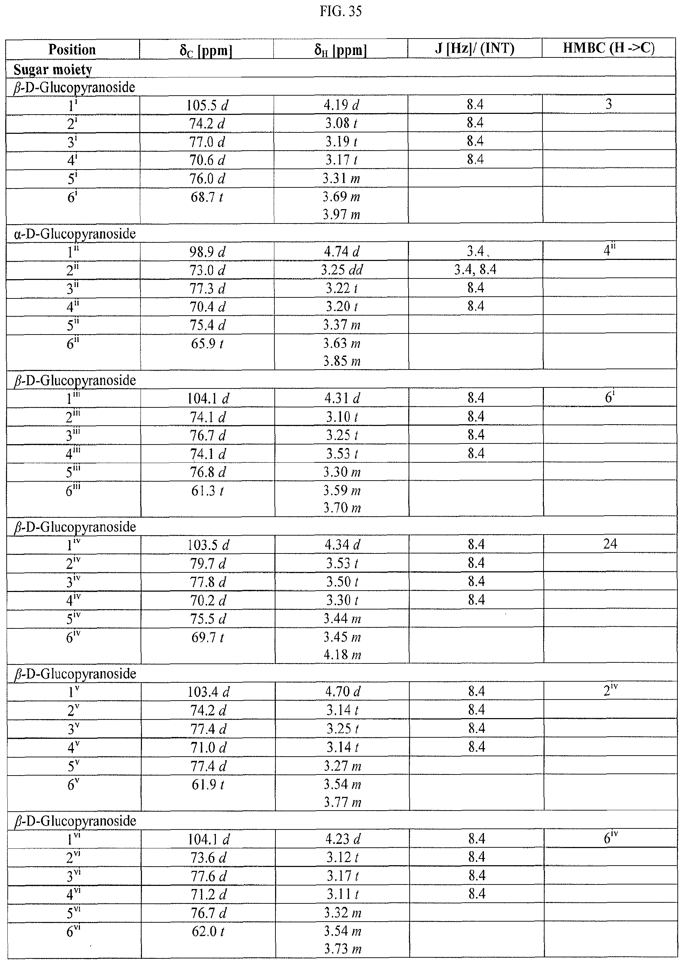

FIG. 35: NMR data of compound 9 (continued)

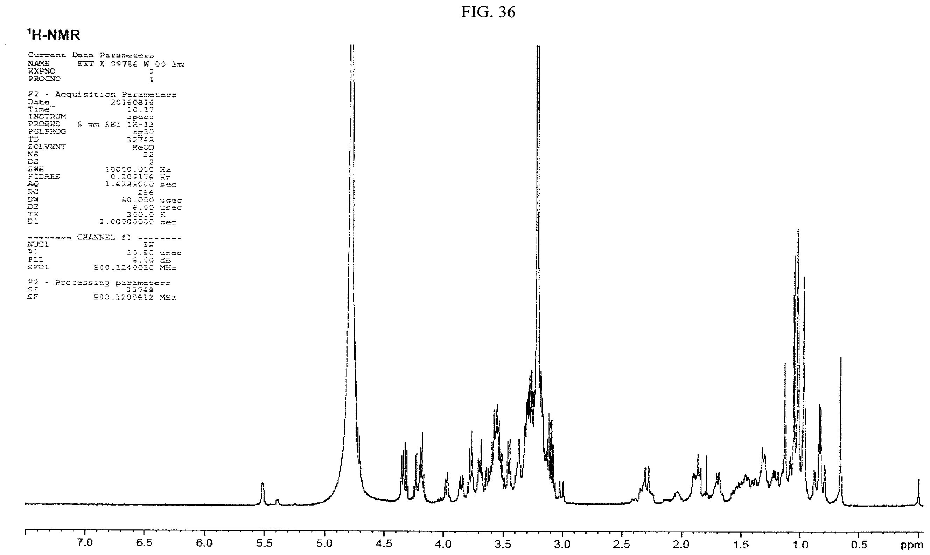

FIG. 36: 1H NMR spectrum of compound 9

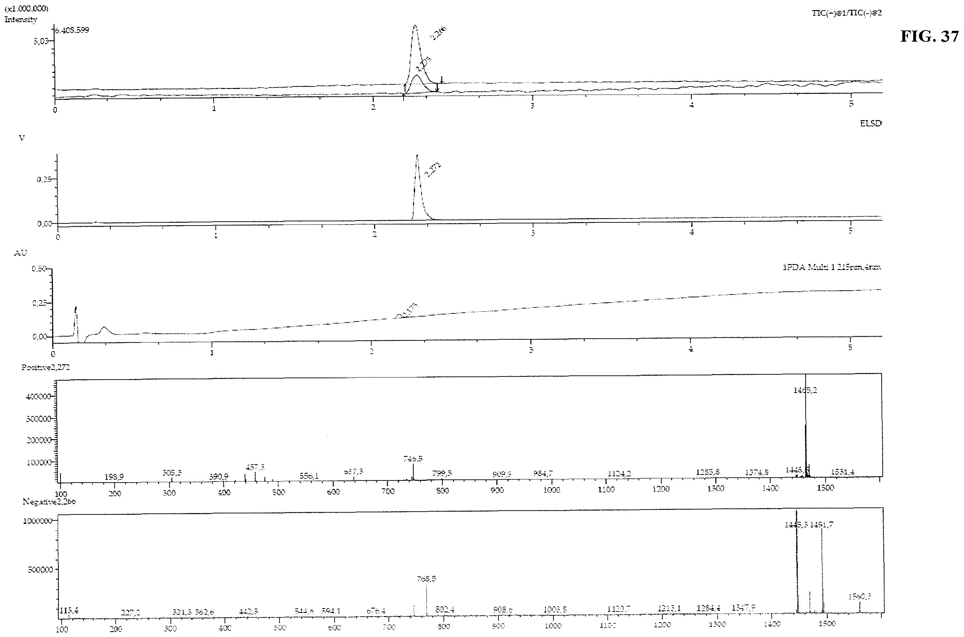

FIG. 37: LCMS of compound 10

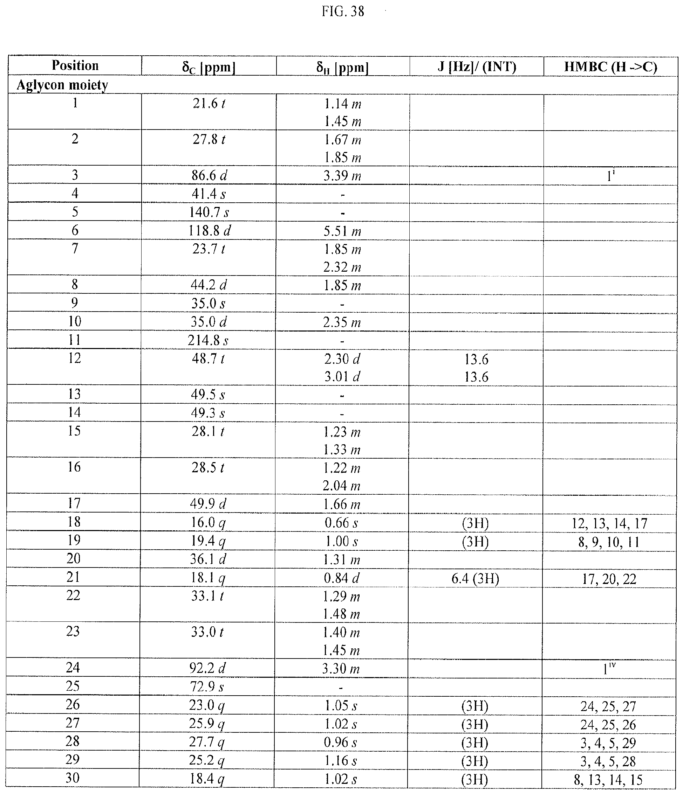

FIG. 38: NMR data of compound 10

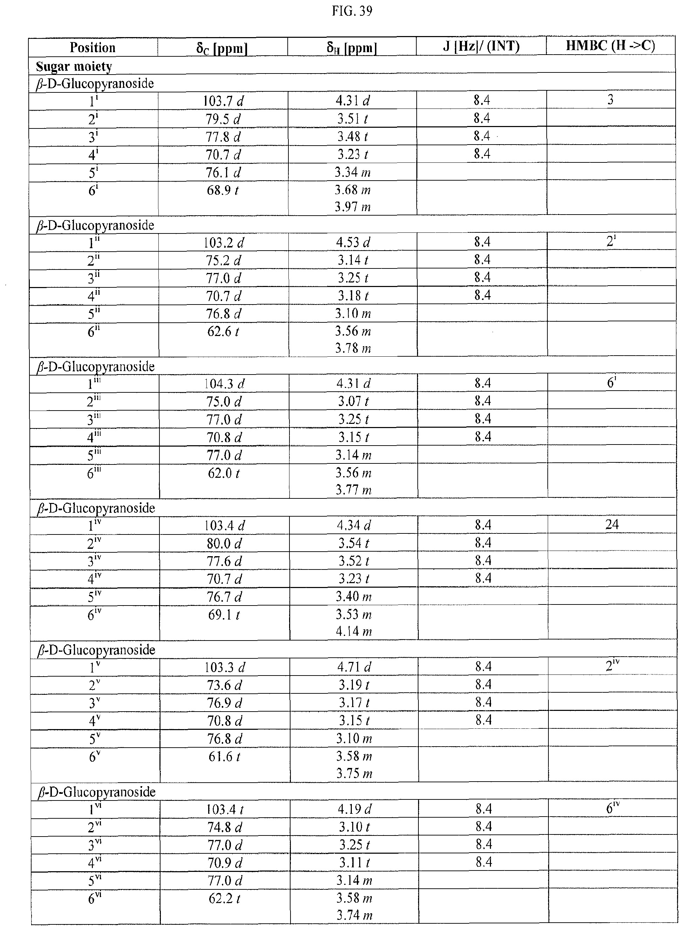

FIG. 39: NMR data of compound 10 (continued)

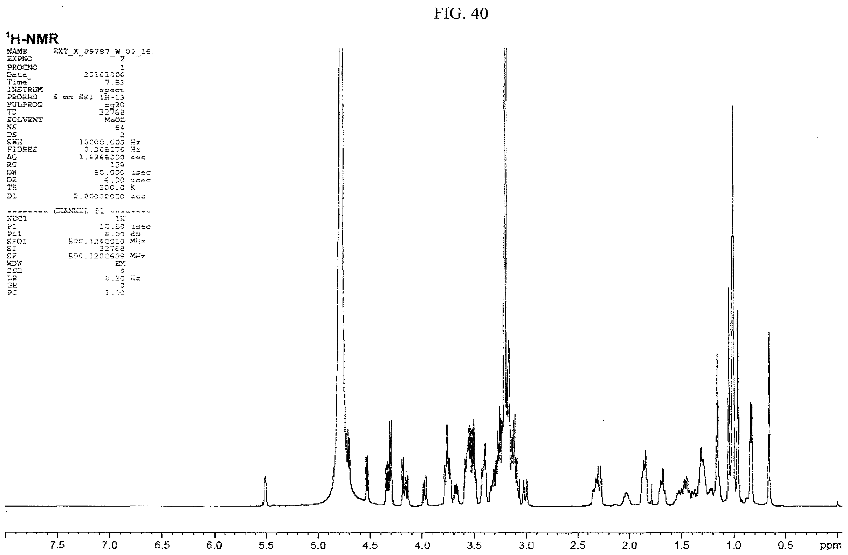

FIG. 40: 1H NMR spectrum of compound 10

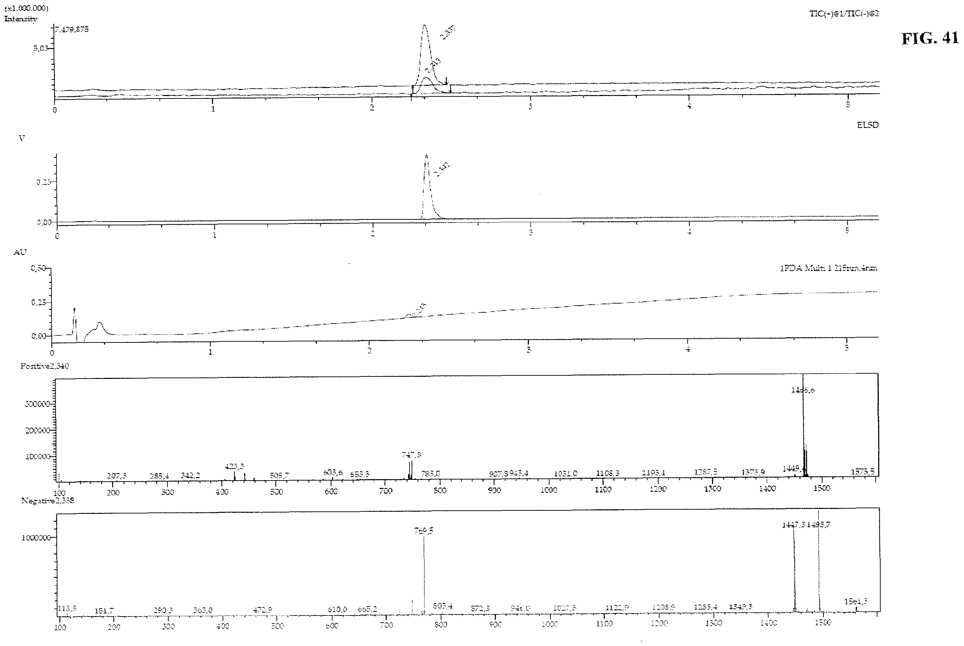

FIG. 41: LCMS of compound 11

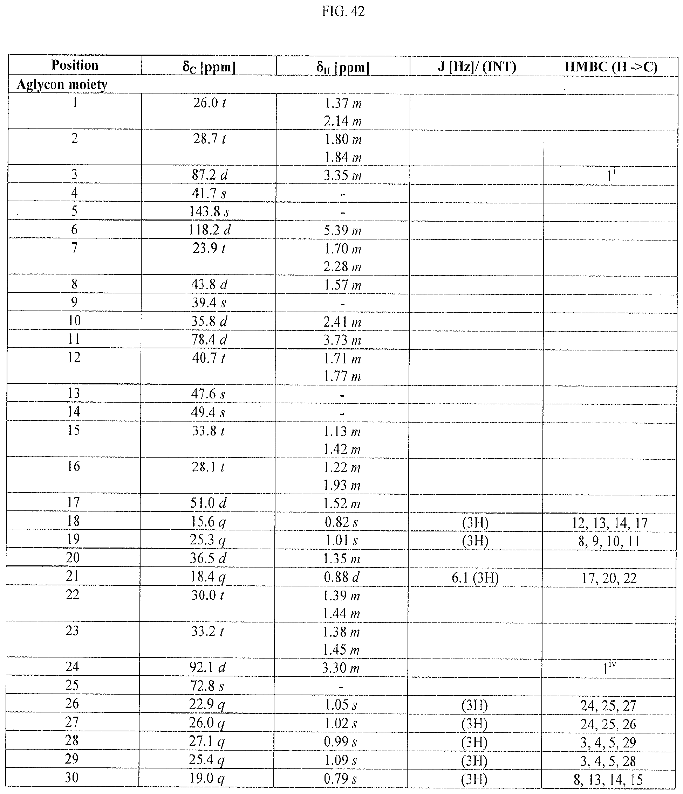

FIG. 42: NMR data of compound 11

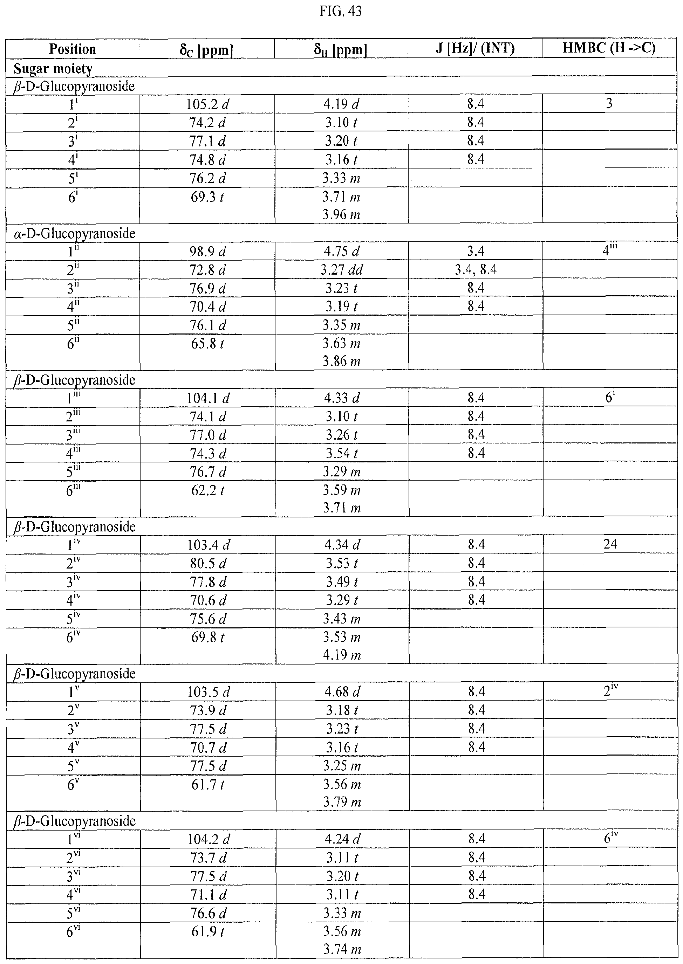

FIG. 43: NMR data of compound 11 (continued)

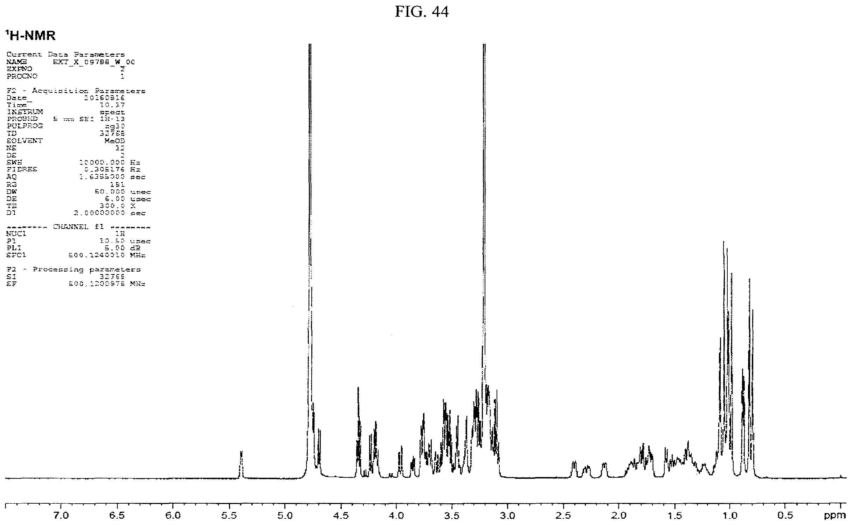

FIG. 44: 1H NMR spectrum of compound 11

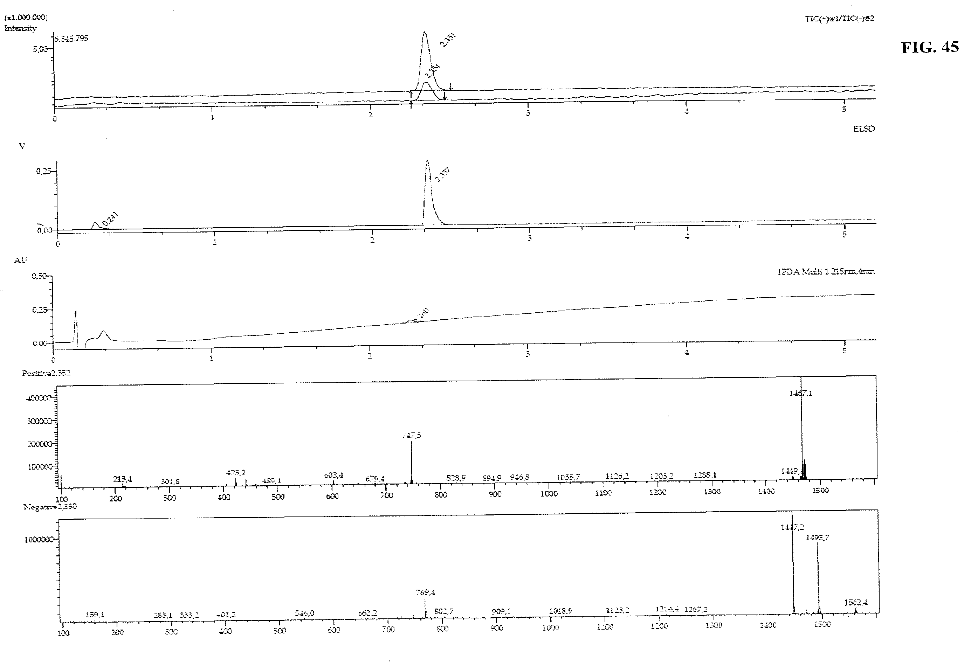

FIG. 45: LCMS of compound 12

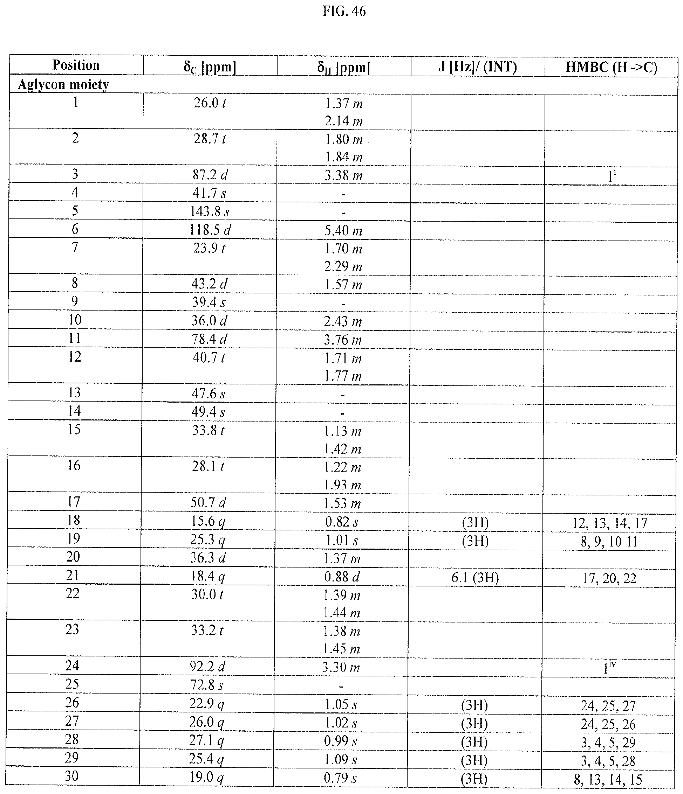

FIG. 46: NMR data of compound 12

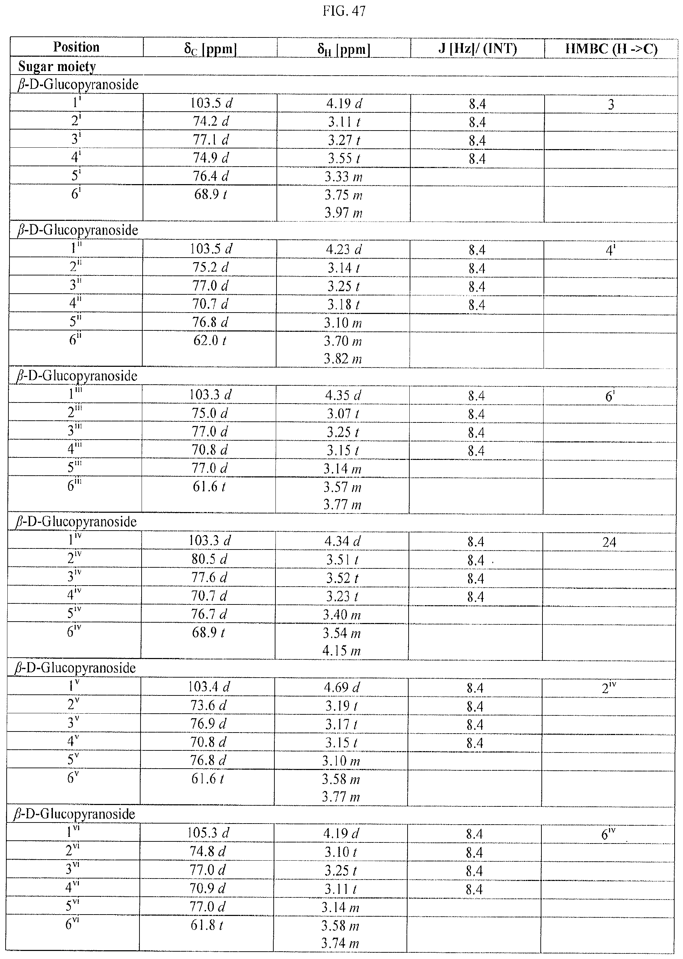

FIG. 47: NMR data of compound 12 (continued)

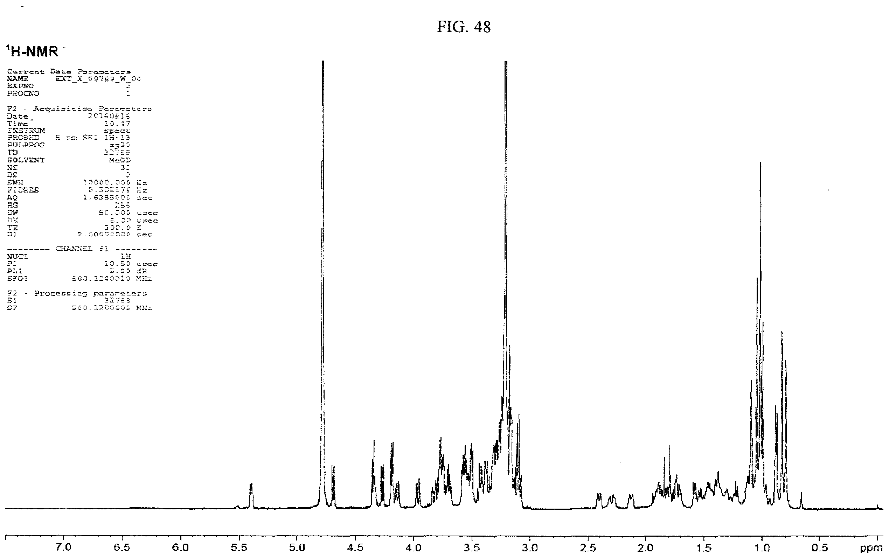

FIG. 48: 1H NMR spectrum of compound 12

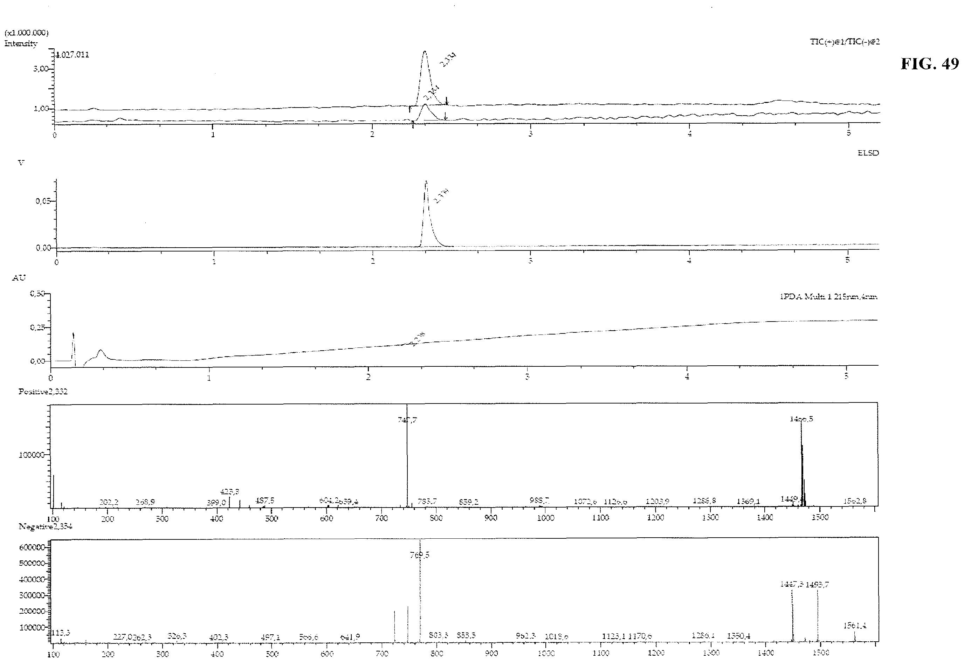

FIG. 49: LCMS of compound 13

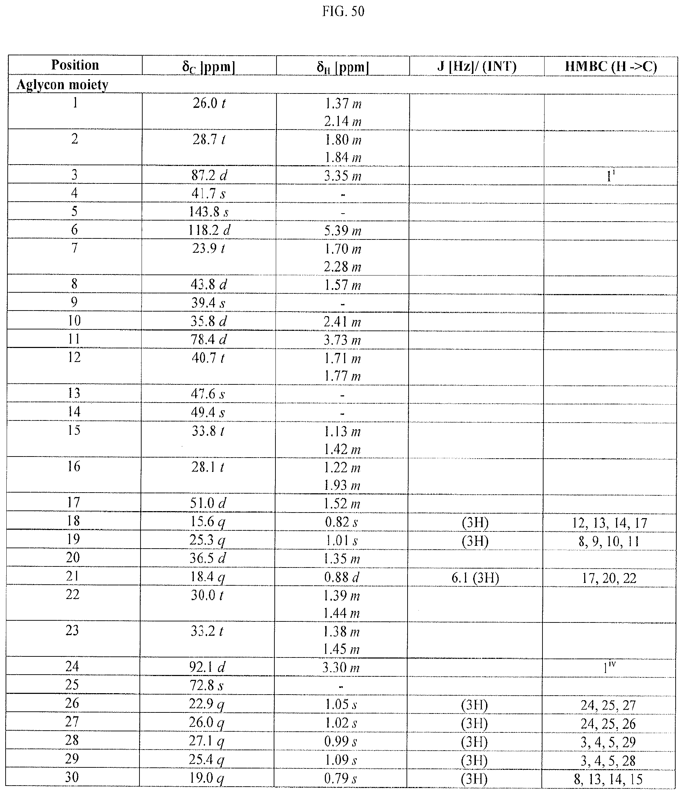

FIG. 50: NMR data of compound 13

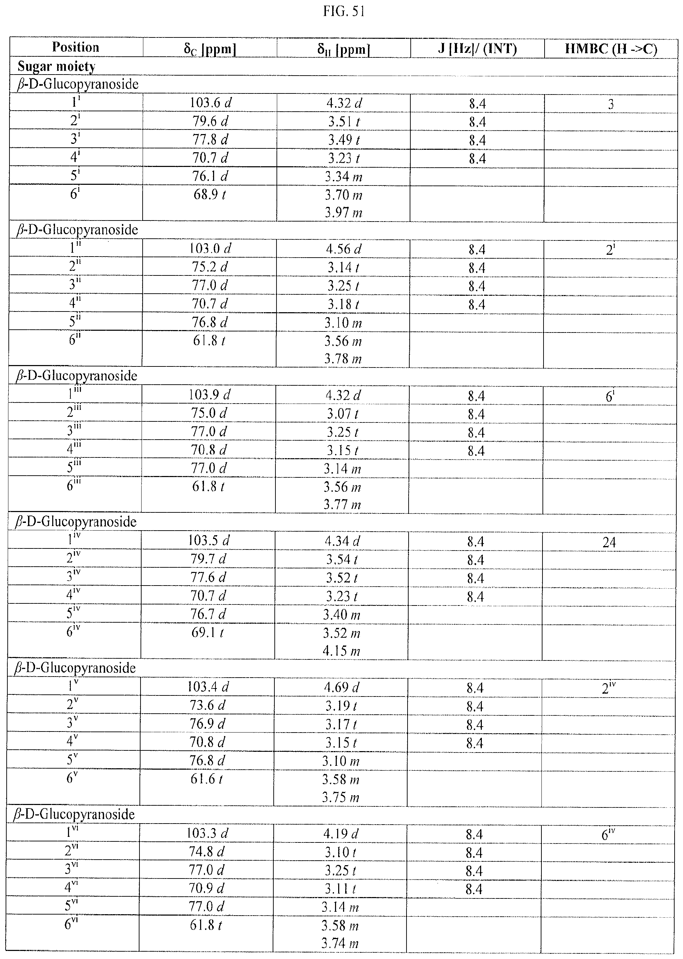

FIG. 51: NMR data of compound 13 (continued)

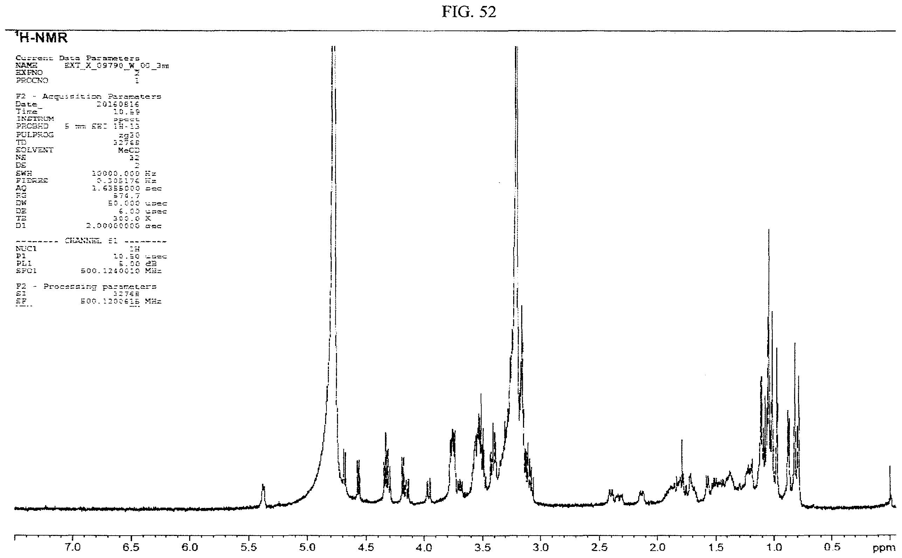

FIG. 52: 1H NMR spectrum of compound 13

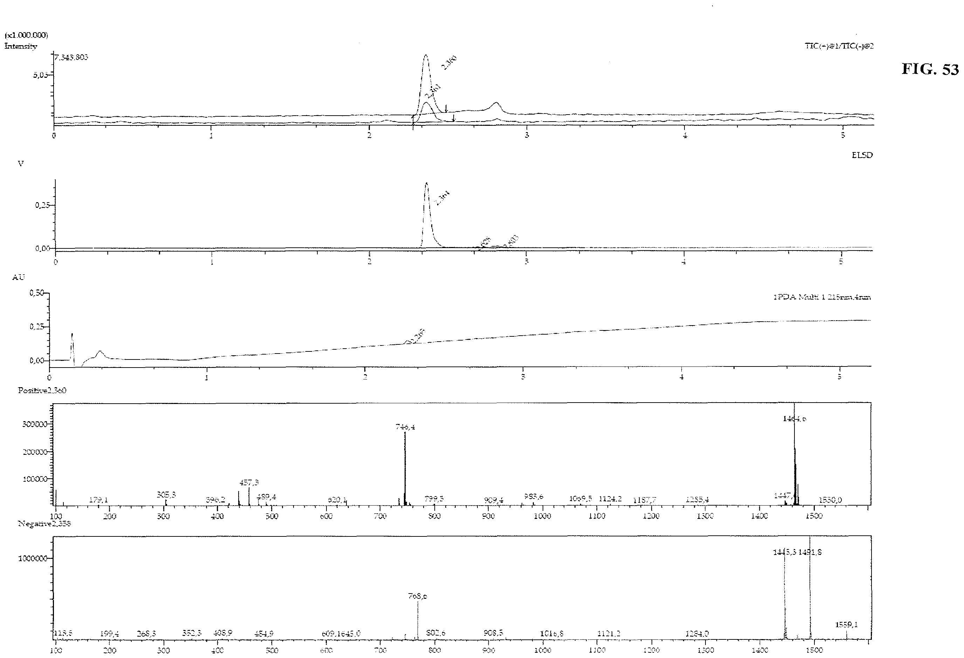

FIG. 53: LCMS of compound 14

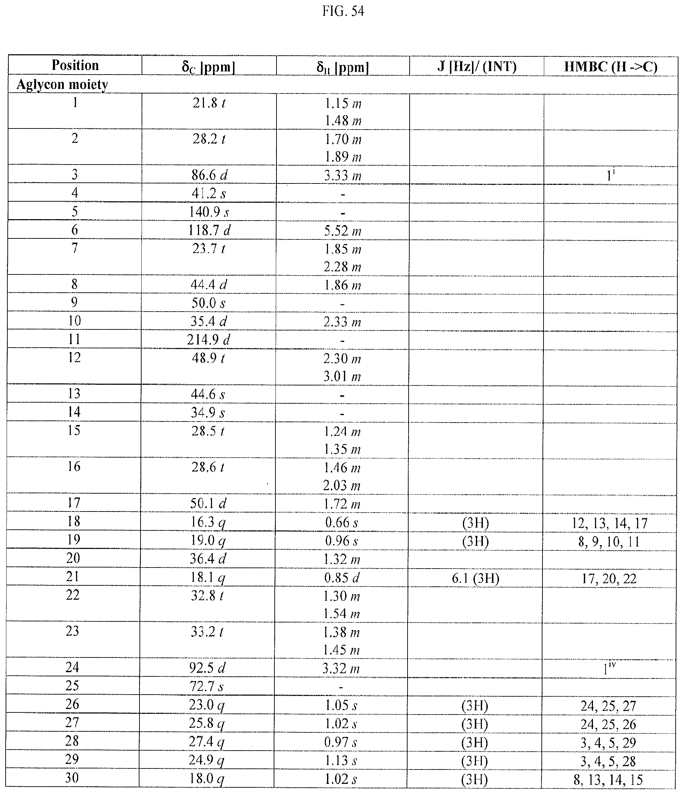

FIG. 54: NMR data of compound 14

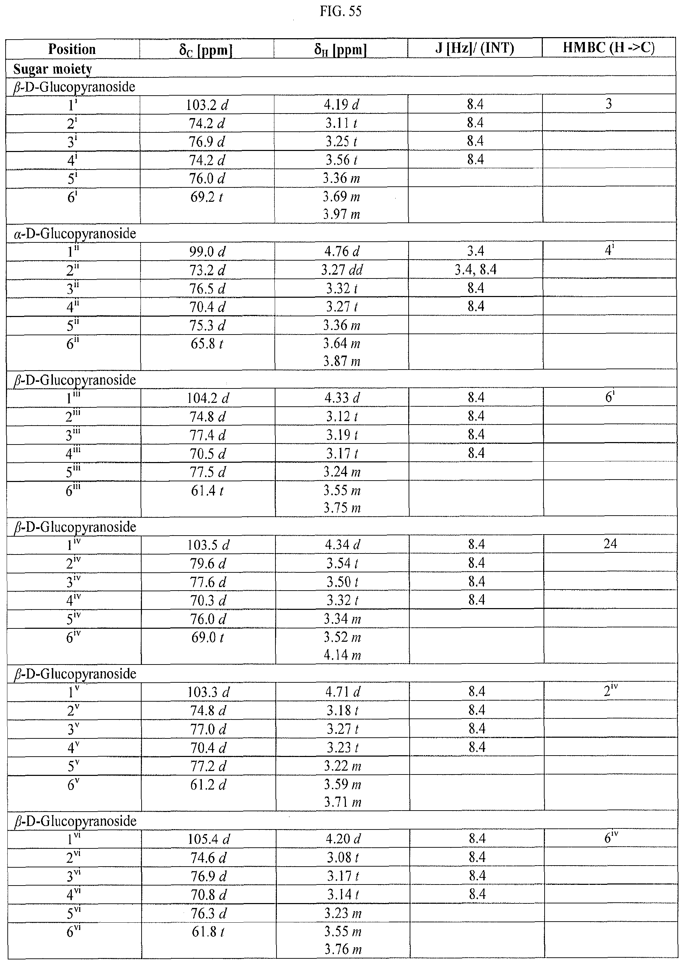

FIG. 55: NMR data of compound 14 (continued)

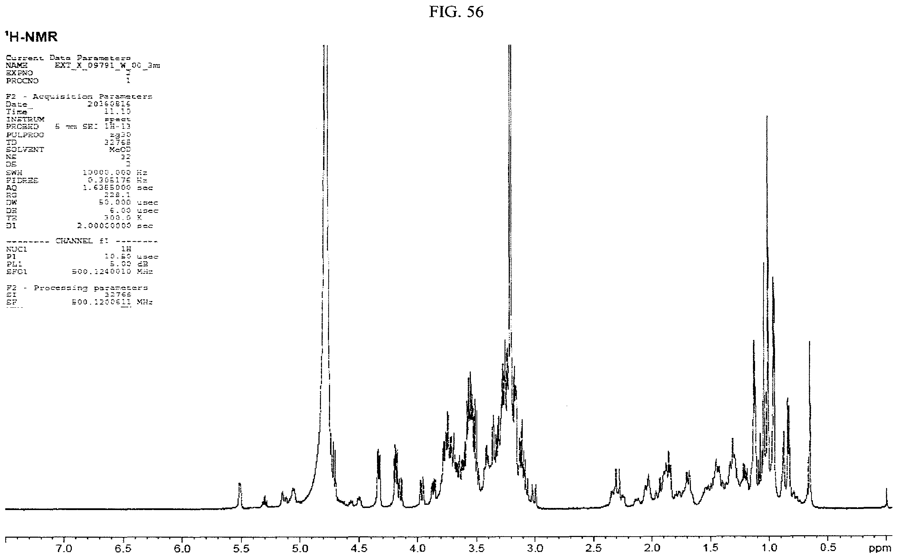

FIG. 56: 1H NMR spectrum of compound 14

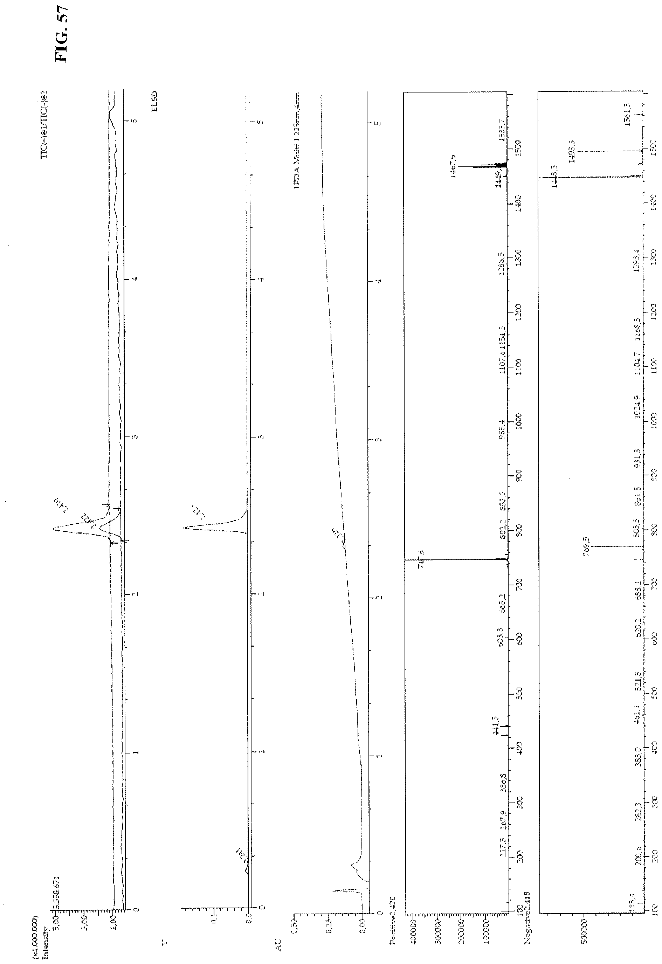

FIG. 57: LCMS of compound 15

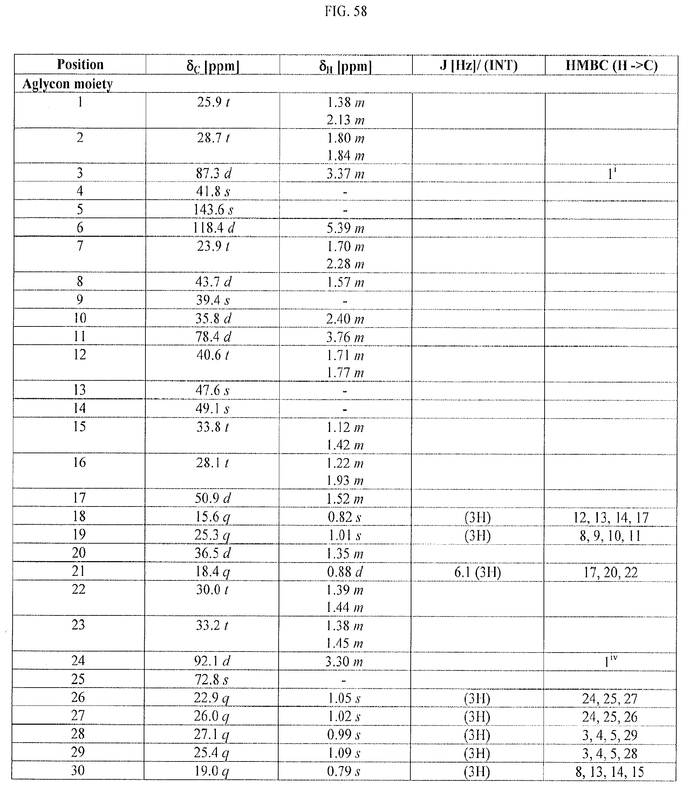

FIG. 58: NMR data of compound 15

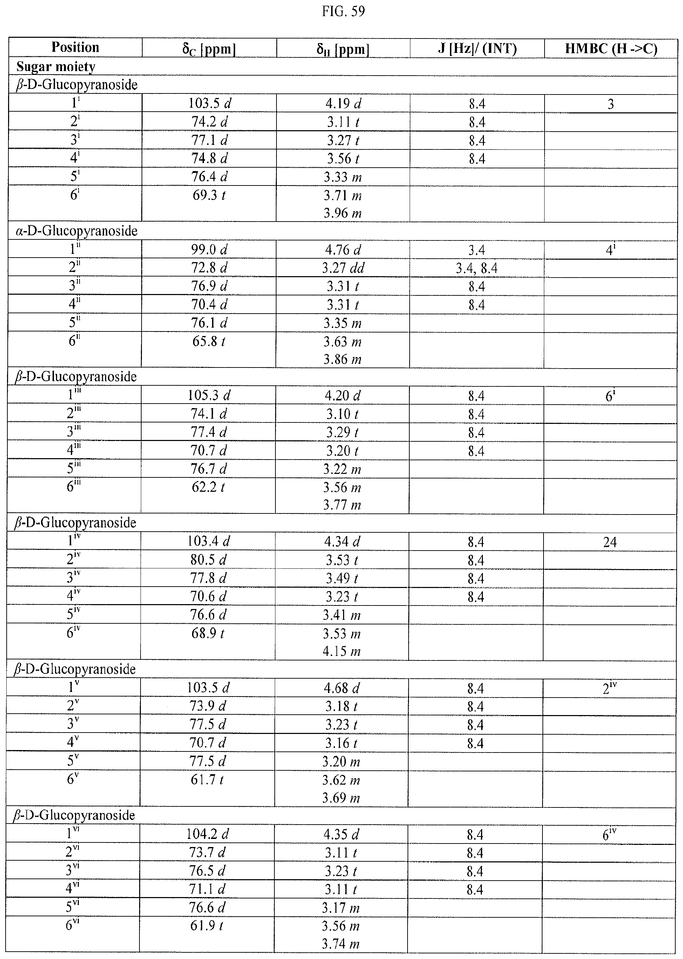

FIG. 59: NMR data of compound 15 (continued)

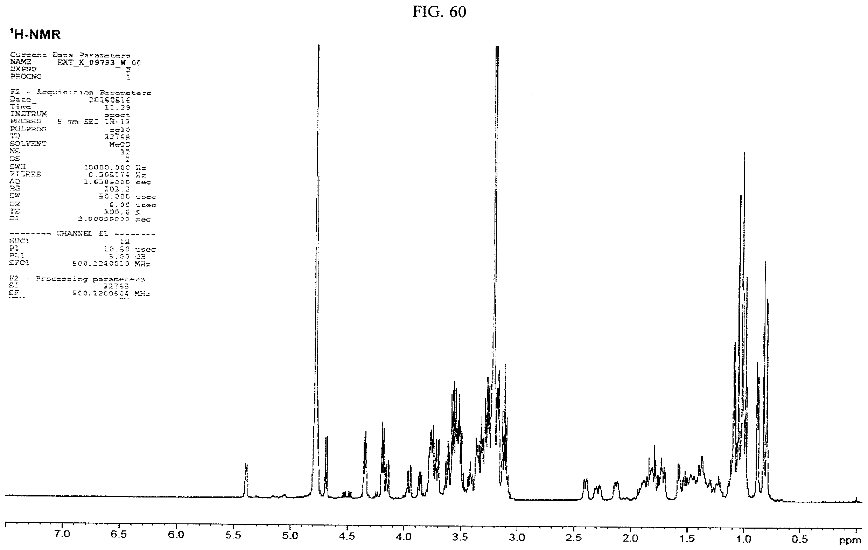

FIG. 60: 1H NMR spectrum of compound 15

### Summary of the Invention

The invention relates to a process for preparation of compositions comprising novel mogrosides from *Siraitia grosvenori* fruit extract. However, this process forms part of the present invention only in as far as it results in any of the compounds (8), (9), (11), (12), (14) or (15) as defined herein.

In one embodiment, the process for purification of novel mogrosides comprises methods described in US Patent Application No. 61/379,729 (filed 09/03/2010), US Patent Application No. 13/219,721 (filed 08/29/2011), now patented as US Patent No. 9,101,162, and US Patent Application No. 14/792,594 (filed 07/06/2015), which was published as US Patent Application Publication No. 2015/0305381.

In yet another embodiment the process further comprises:
a. providing a mixture of mogrosides;
b. dissolving the mogrosides mixture in solvent to form an initial solution of mogrosides;
c. passing the initial solution through a chromatographic system,
d. separating the fractions comprising novel mogroside compounds 1-15.

The present invention further provides compositions comprising novel mogroside compounds (8), (9), (11), (12), (14) or (15), as defined herein.

In one embodiment the compositions further comprise another high intensity sweetener selected from the group consisting of stevioside, rebaudioside A, rebaudioside B, rebaudioside C, rebaudioside D, rebaudioside E, rebaudioside F, rebaudioside G, rebaudioside H, rebaudioside I, rebaudioside J, rebaudioside K, rebaudioside L, rebaudioside M, rebaudioside N, rebaudioside O, dulcoside A, dulcoside B, rubusoside, other steviol glycosides occurring in *Stevia rebaudiana* plant, glycosylated steviol glycosides, biosynthetic steviol glycosides, Luo Han Guo sweetener, siamenoside, mogroside IV, mogroside V, mogroside VI, other mogrosides occurring in *Siraitia grosvenorii* fruits, monatin and its salts (monatin SS, RR, RS, SR), glycyrrhizic acid and its salts, curculin, thaumatin, monellin, mabinlin, brazzein, hernandulcin, phyllodulcin, glycyphyllin, phloridzin, trilobatin, baiyunoside, osladin, polypodoside A, pterocaryoside A, pterocaryoside B, mukurozioside, phlomisoside I, periandrin I, abrusoside A, cyclocarioside I, and combinations thereof.

The present invention also provides consumables comprising novel mogroside compounds (8), (9), (11), (12), (14) or (15), as defined herein.

In one embodiment the consumable is selected from the group consisting of food, beverage, pharmaceutical composition, tobacco, nutraceutical, oral hygienic composition, or cosmetic.

### DETAILED DESCRIPTION OF THE INVENTION

Advantages of the present invention will become more apparent from the detailed description given hereinafter. However, it should be understood that the detailed description and specific examples, while indicating preferred embodiments of the invention, are given by way of illustration only, the present invention being defined in the claims.

The present invention provides a process for purification of novel mogroside compounds (8), (9), (11), (12), (14) or (15) from *Siraitia grosvenorii* fruit.

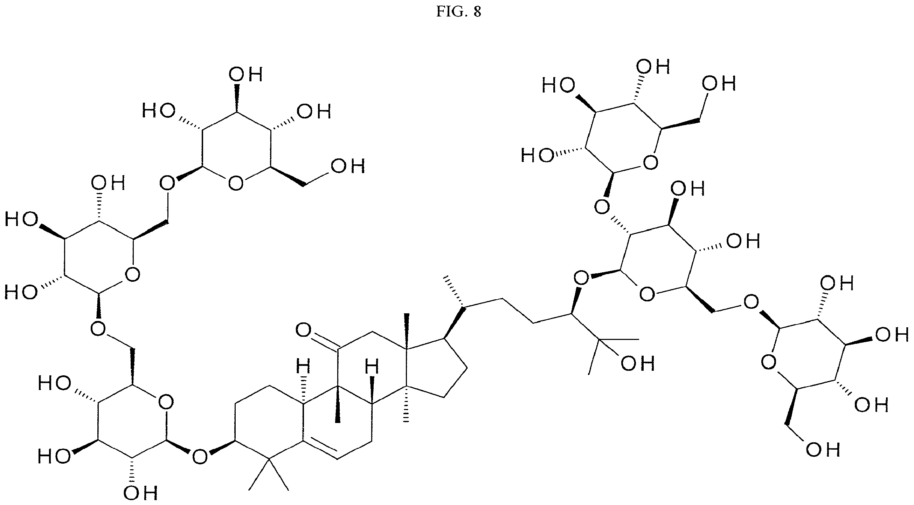

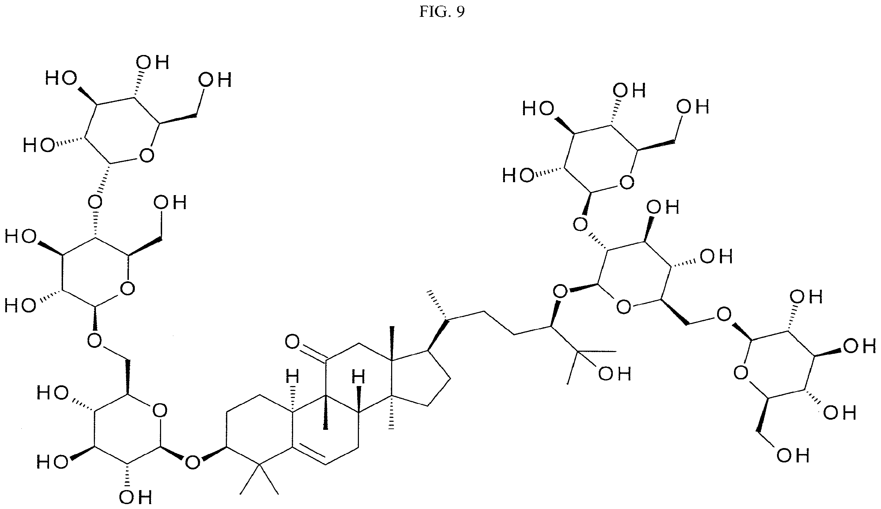

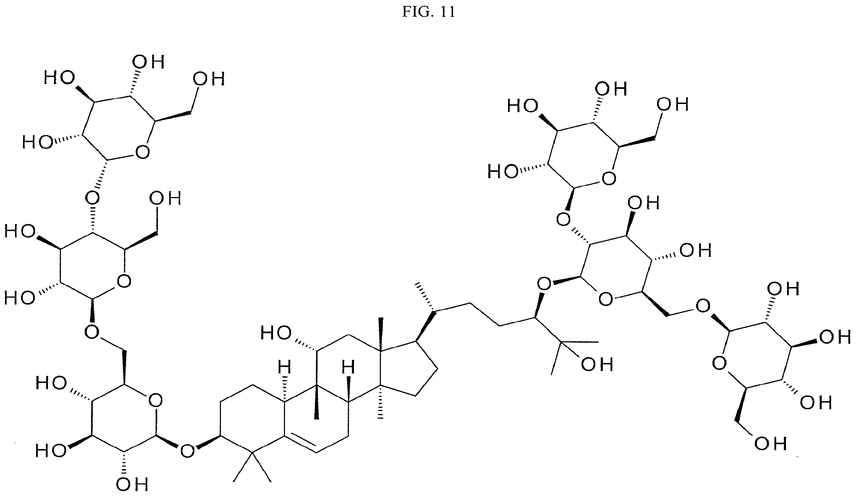

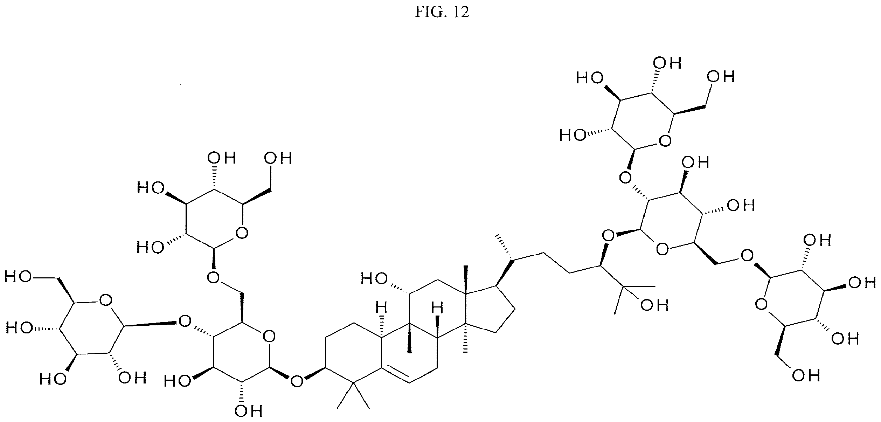

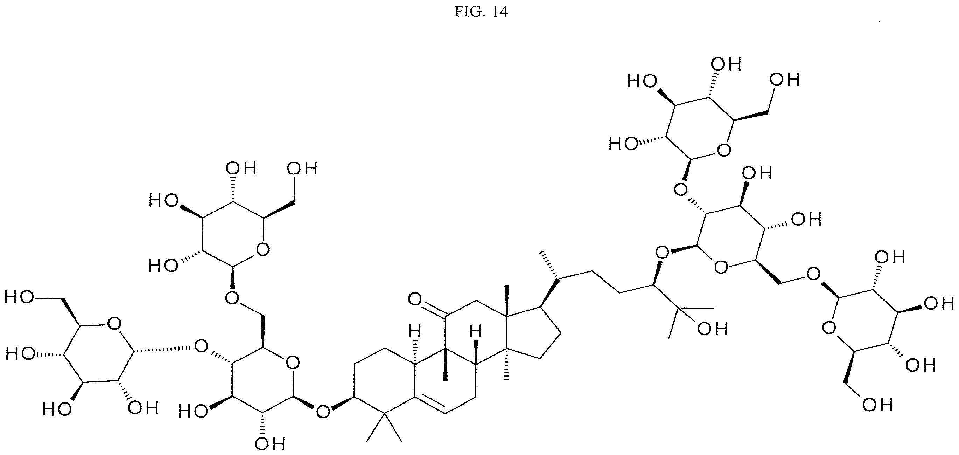

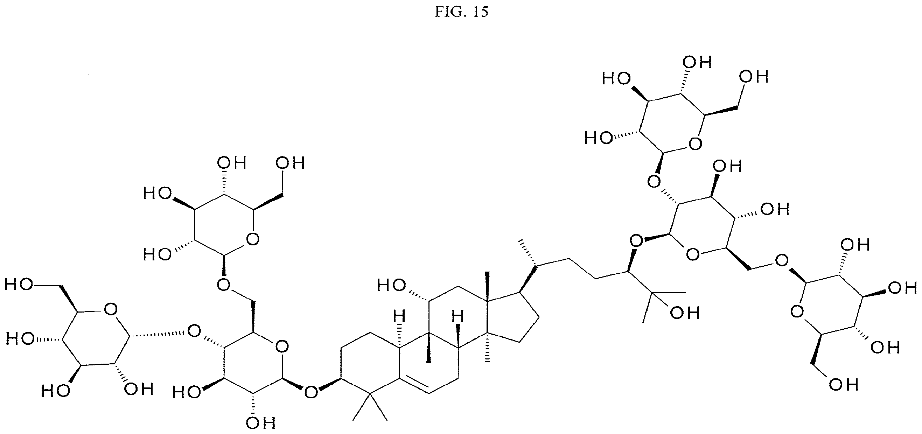

Hereinafter the term "novel mogroside(s)" refers to compound(s) (8), (9), (11), (12), (14) and (15) (-FIG. 8, 9, 11, 12, 14 and 15 respectively).

The process of purification of novel mogrosides of the present invention comprises methods described in US Patent Application No. 61/379,729 (filed 09/03/2010), US Patent Application No. 13/219,721 (filed 08/29/2011), now patented as US Patent No. 9,101,162, and US Patent Application No. 14/792,594 (filed 07/06/2015), which was published as US Patent Application Publication No. 2015/0305381.

The process of the present invention may further comprise isolation and purification of novel mogrosides using chromatography system.

In one embodiment of present invention a solution comprising at least one novel mogroside was passed through a HPLC column and fractions comprising novel mogrosides were collected. The HPLC column can be any suitable HPLC analytical or preparative scale column. The fractions may be eluted by adding an appropriate eluent. The eluent can be any suitable solvent or combination of solvents. In one embodiment, the eluent is water and/or acetonitrile. The method may optionally comprise additional steps, such as removal of solvents from the eluted solution to provide a concentrate comprising at least one novel mogroside.

In one embodiment, the following chromatography systems were used for isolation and purification of novel mogrosides. The mogrosides sample from a natural source was subjected to preparative HPLC using a commercial polyamine column, eluting with a mobile phase comprising acetonitrile and water. The mogrosides fraction from this step was further subjected to preparative HPLC using a commercial C18 column, eluting with a mobile phase comprising acetonitrile and water. Individual fractions from this step were further subjected to analytical-preparative HPLC using a commercial C18 column, eluting with a mobile phase comprising acetonitrile and water. The results of isolation and purification are summarized in Table 2. The purity of the isolated mogroside samples was suitable for NMR and HPLC-MS analysis.

**Table 2**

| The present invention relates to compounds (8), (9), (11), (12), (14) and (15). Any other compounds referred to herein are for the purposes of comparison only. | | | |
|---|---|---|---|
| Column | Loading Material | Fractions collected | Novel Mogroside compound(s) |
| Polyamine | Commercial Mogrosides extract | 1 combined fraction | Compounds 8-15 |
| Preparative C18 | Fraction from Polyamine column | Fraction 1 | Compounds 8 + 9 |
| | | Fraction 2 | Compounds 10 + 11 |
| | | Fraction 3 | Compound 12 |
| | | Fraction 4 | Compounds 13 + 14 |
| | | Fraction 5 | Compound 15 |
| Analytical Preparative C18 | Fraction 1 | Individual peak | Compound 8 |
| | | Individual peak | Compound 9 |
| | Fraction 2 | Individual peak | Compound 10 |
| | | Individual peak | Compound 11 |
| | Fraction 4 | Individual peak | Compound 13 |
| | | Individual peak | Compound 14 |

In one embodiment, the fractions comprising novel mogrosides were dried to produce compositions comprising at least one novel mogroside.

The compositions comprising at least one novel mogroside can be also produced from new cultivars of *Siraitia grosvenorii* plant which have increased content of novel mogrosides.

The compositions comprising at least one novel mogroside can be also produced by fermentation of recombinant microbial hosts selected from genera *Candida, Cyberlindnera, Kluyveromyces, Meyerozyma, Pischia., Rhodosporidium, Zygosaccharomyces, Saccharomyces, Aspergillus, Hansenula, Humicola, Trichosporon, Brettanomyces, Pachzysolen, Yarrowia, Yamadazyma, Schizosaccharomyces, Ashbya, Cyberlindnera, Pichia, Arxula, Xanthophyllomyces or Escherichia.* Non-limiting examples of microbial strains include *Arthrobacter globiformis, Aspergillus niger, Aspergillus oryzae, Bacillus licheniformis, Bacillus sphaericus, Bacillus subtilis, Brevibacterium linens, Candida utilis, Candida vini, Corynebacterium glutamicum, Cyberlindnera jadinii, Cyberlindnera* sp., *Debaryomyces hansenii, Fusarium semitectum, Hypomyces αrmeniacus, Kluyveromyces lactis, Kluyveromyces marxianus, Kocuria rhizophila, Lactobacillus brevis, Lactobacillus casei, Lactobacillus pentoses, Lactobacillus plantarum, Lactobacillus reuteri, Meyerozyma guilliermondii, Microbacterium* sp., *Micrococcus luteus, Mucor hiemalis, Mucor racemosus, Penicillium roqueforti, Pichia guilliermondii, Pichia jadinii, Pichia pastoris, Pseudomonas fluorescens, Pseudomonas stutzeri, Rhodosporidium* sp., *Rhodosporidium toruloides, Rhodotorula mucilaginosa, Rhodotorula rubra, Rhodotorula* sp., *Saccharomyces bayanus, Saccharomyces cerevisiae, Saccharomyces pastorianus, Streptomyces albus, Streptomyces coelicolor, Streptomyces griseus, Streptomyces lividans, Torulaspora delbrueckii, Trichosporon laibachii, Trichosporon oleaginosus, Yarrowia lipolytica, Zygosaccharomyces rouxii, Zymomonas mobilis.*

The compositions comprising at least one novel mogroside can be also produced by biotransformation using suitable biocatalysts.

The compositions comprising at least one novel mogroside can be also produced by chemical synthesis from precursor compounds.

One embodiment of present invention is a composition comprising at least one novel mogroside.

In some embodiments, at least one novel mogroside imparts sweet taste.

In one embodiment, the present invention is a sweetener composition comprising at least one novel mogroside.

In another embodiment, the present invention is a flavor-enhancing composition comprising at least one novel mogroside, wherein the novel mogroside is present in an amount effective to provide a concentration at or below the threshold flavor recognition level of the novel mogroside when the flavor-enhancing composition is added to a consumable. In a particular embodiment, the novel mogroside is present in an amount effective to provide a concentration below the threshold flavor recognition level of the novel mogroside when the flavor-enhancing composition is added to a consumable. In one embodiment, the novel mogroside is present in an amount effective to provide a concentration at least about 1%, at least about 5%, at least about 10%, at least about 15,% at least about 20% or at least about 25% or more below the threshold flavor recognition level of the novel mogroside when the flavor-enhancing composition is added to a consumable.

In yet another embodiment, the present invention is a sweetness-enhancing composition comprising at least one novel mogroside, wherein the novel mogroside is present in an amount effective to provide a concentration at or below the threshold sweetness recognition level of the novel mogroside when the sweetness-enhancing composition is added to a consumable. In a particular embodiment, the novel mogroside is present in an amount effective to provide a concentration below the threshold sweetness recognition level of the novel mogroside when the sweetness-enhancing composition is added to a consumable. In one embodiment, the novel mogroside is present in an amount effective to provide a concentration at least about 1%, at least about 5%, at least about 10%, at least about 15,% at least about 20% or at least about 25% or more below the threshold sweetness recognition level of the novel mogroside when the sweetness-enhancing composition is added to a consumable.

In yet another embodiment, the present invention is a consumable comprising at least one novel mogroside. Suitable consumables include, but are not limited to, liquid-based or dry consumables, such as, for example, pharmaceutical compositions, edible gel mixes and compositions, dental compositions, foodstuffs, beverages and beverage products.

In a particular embodiment, the present invention is a beverage comprising a at least one novel mogroside. In a particular embodiment, the novel mogroside is present in the beverage at a concentration that is above, at or below the threshold sweetness recognition concentration of the novel mogroside.

In another particular embodiment, the present invention is a beverage product comprising a at least one novel mogroside. In a particular embodiment, the novel mogroside is present in the beverage product at a concentration that is above, at or below the threshold flavor recognition concentration of the novel mogroside.

In another aspect, the present invention is a method of preparing a consumable comprising (i) providing a consumable matrix and (ii) adding at least one novel mogroside to the consumable matrix to provide a consumable. In a particular embodiment, the novel mogroside is present in the consumable in a concentration above, at or below the threshold sweetness recognition of the novel mogroside. In another particular embodiment, the novel mogroside is present in the consumable in a concentration above, at or below the threshold flavor recognition of the novel mogroside.

In a particular embodiment, the present invention is a method of preparing a beverage comprising (i) providing a beverage matrix and (ii) adding at least one novel mogroside to the consumable matrix to provide a beverage. In a particular embodiment, the novel mogroside is present in the consumable in a concentration above, at or below the threshold sweetness recognition of the novel mogroside. In another particular embodiment, the novel mogroside is present in the consumable in a concentration above, at or below the threshold flavor recognition concentration of the novel mogroside.

In another aspect, the present invention is a method of enhancing the sweetness of a consumable comprising (i) providing a consumable comprising at least one sweet ingredient and (ii) adding at least one novel mogroside to the consumable to provide a consumable with enhanced sweetness, wherein the novel mogroside is present in the beverage with enhanced sweetness at a concentration at or below the threshold sweetness recognition concentration of the novel mogroside.

In a particular embodiment, the present invention is a method of enhancing the sweetness of a beverage comprising (i) providing a beverage comprising at least one sweet ingredient and (ii) adding at least one novel mogroside to the beverage to provide a beverage with enhanced sweetness, wherein the novel mogroside is present in the beverage with enhanced sweetness at a concentration below the threshold sweetness recognition concentration of the novel mogroside. In one embodiment, the concentration of the novel mogroside is present in the beverage with enhanced sweetness at a concentration that is at least about 1%, at least about 5%, at least about 10%, at least about 15%, at least about 20%, or at least about 25% or more below the threshold sweetness recognition concentration of the novel mogroside.

In a further aspect, the present invention is a method of enhancing the flavor of a consumable comprising (i) providing a consumable comprising at least one flavor ingredient and (ii) adding at least one novel mogroside to the consumable to provide a consumable with enhanced flavor, wherein the novel mogroside in present in the consumable with enhanced flavor at a concentration at or below the threshold flavor recognition concentration of the novel mogroside.

In a particular embodiment, the present invention is a method of enhancing the flavor of a beverage comprising (i) providing a beverage comprising at least one flavor ingredient and (ii) adding at least one novel mogroside to the beverage to provide a beverage with enhanced flavor, wherein the novel mogroside is present in the beverage with enhanced flavor in a concentration at or below the threshold flavor recognition concentration of the novel mogroside. In one embodiment, the concentration of the novel mogroside is present in the beverage with enhanced sweetness at a concentration that is at least about 1%, at least about 5%, at least about 10%, at least about 15%, at least about 20%, or at least about 25% or more below the threshold flavor recognition concentration of the novel mogroside.

In the above methods, the novel mogroside may be added as such, or in the form of a composition comprising the novel mogroside. When the novel mogroside is provided as a composition, the concentration of the novel mogroside in the composition is effective to provide a concentration above, at or below the threshold flavor or sweetener composition of the novel mogroside, when the composition is added to the consumable, e.g., the food or beverage.

In some embodiments, the compositions of the present invention comprise one or more additional mogrosides, where the additional mogrosides are selected from, but not limited to, the group consisting of Luo han guo extract, by-products of other mogrosides' isolation and purification processes, a commercially available Luo han guo extract, mogroside IIE, mogroside IIB, mogroside III, mogroside IV, mogroside V, 11-oxo-mogroside V, mogroside VI, siamenoside I, grosmomoside I, neomogroside, and other mogrol and oxo-mogrol glycosides occurring in *Siraitia grosvenorii* fruit and combinations thereof.

In other embodiments, the compositions of the present invention comprise one or more sweeteners or additional sweeteners. In one embodiment, the additional sweetener is a natural sweetener or a synthetic sweetener. In a particular embodiment, the additional sweetener is a high intensity sweetener. In a particular embodiment, the additional sweetener is a mogroside.

In some embodiments, the compositions of the present invention comprise one or more additives. In a particular embodiment, the additive is selected from the group consisting of carbohydrates, polyols, amino acids and their corresponding salts, poly-amino acids and their corresponding salts, sugar acids and their corresponding salts, nucleotides, organic acids, inorganic acids, organic salts including organic acid salts and organic base salts, inorganic salts, bitter compounds, flavorants and flavoring ingredients, astringent compounds, proteins or protein hydrolysates, surfactants, emulsifiers, flavonoids, alcohols, polymers and combinations thereof.

In some embodiments, the compositions of the present invention comprise one or more functional ingredients. In a particular embodiment, the functional ingredient is selected from the group consisting of caffeine, saponins, antioxidants, dietary fiber sources, fatty acids, vitamins, glucosamine, minerals, preservatives, hydration agents, probiotics, prebiotics, weight management agents, osteoporosis management agents, phytoestrogens, long chain primary aliphatic saturated alcohols, phytosterols and combinations thereof.

In a particular embodiment, the present invention is a consumable comprising a novel mogroside and one or more additional mogrosides, sweeteners, additional sweeteners, additives or functional ingredients.

In another particular embodiment, the present invention is a beverage comprising at least one novel mogroside and one or more additional mogrosides, sweeteners, additional sweeteners, additives or functional ingredients.

The compositions comprising novel mogrosides can be used either alone or in combination with at least one other sweetener in consumables including food, beverage, pharmaceutical composition, tobacco, nutraceutical, oral hygienic composition, or cosmetic. The other sweeteners are selected from the group including sucrose, glyceraldehyde, dihydroxyacetone, erythrose, threose, erythrulose, arabinose, lyxose, ribose, xylose, ribulose, xylulose, allose, altrose, allulose, galactose, glucose, gulose, idose, mannose, talose, fructose, psicose, sorbose, tagatose, mannoheptulose, sedoheltulose, octolose, fucose, rhamnose, arabinose, turanose, sialose, inulin, inulooligosaccharides, fructooligosaccharides, high fructose corn syrup (HFCS), maltodextrin, coupling sugar, honey, stevia, rebaudioside A, rebaudioside B, rebaudioside C, rebaudioside D, rebaudioside E, rebaudioside F, rebaudioside G, rebaudioside H, rebaudioside I, rebaudioside J, rebaudioside K, rebaudioside L, rebaudioside M, rebaudioside N, rebaudioside O, dulcoside A, dulcoside B, rubusoside, steviolbioside, stevioside, other steviol glycosides occurring in *Stevia rebaudiana* plant, biosynthetic steviol glycosides, glycosylated steviol glycosides, glucosylated steviol glycosides (GSGs), mogroside IV, mogroside V, mogroside VI, Luo han guo, siamenoside, other mogrosides occurring in *Siraitia grosvenorii* fruits, monatin and its salts, curculin, glycyrrhizic acid and its salts, thaumatin, monellin, mabinlin, brazzein, hernandulcin, phyllodulcin, glycyphyllin, phloridzin, trilobatin, baiyunoside, osladin, polypodoside A, pterocaryoside A, pterocaryoside B, mukurozioside, phlomisoside I, periandrin I, abrusoside A, and cyclocarioside I, sugar alcohols, sucralose, potassium acesulfame, acesulfame acid and salts thereof, aspartame, alitame, saccharin and salts thereof, neohesperidin dihydrochalcone, cyclamate, cyclamic acid and salts thereof, neotame, advantame, and combinations thereof.

The following examples are provided for illustrating the process and column system of the present invention.

### EXAMPLE 1

### Purification of novel mogrosides

Luo Han Guo fruit extract, commercialized by PureCircle Sdn Bhd (Malaysia) containing 50.72% Mogroside V, 8.58% 11-oxo-Mogroside V, 5.67% Siamenoside I, 5.28% Grosmomoside I and about 3% of mogrosides 1-15 was separated by a column system described in US 61/379,729, US 9,101,162 and US14/792,594 to obtain an enriched composition comprising about 20% of mogrosides 1-15, 31.1 % Mogroside V, 2.8% 11-oxo-Mogroside V, 1.6% Siamenoside I, and 1.1% Grosmomoside I, all percentages being on w/w dried basis. The enriched composition was concentrated to approximately 50% solids content and subjected to preparative HPLC using YMC Polyamine II column (250 X 20mm; 5µm) at ambient temperature, eluting with a mobile phase consisting of acetonitrile and water with 75:25 (vol/vol) ratio (23 mL/min flowrate).

The mogrosides fraction from the preceding step was concentrated to about 7% solids content and was further subjected to preparative HPLC using Zorbax SB-C18 (9.4 X 50 mm; 5µm) column, eluting with a mobile phase consisting of acetonitrile and water with 14:86 vol/vol ratio (35 mL/min flowrate). Five fractions were collected (Table 2) and analysed by analytical-preparative HPLC for purity assessment. Fraction 1 contained two peaks corresponding to mogroside compounds 8 and 9, fraction 2 contained two peaks corresponding to mogroside compounds 10 and 11, fraction 3 contained one major peak corresponding to mogroside compound 12, fraction 4 contained at least two peaks corresponding to mogroside compound 13 and 14, and fraction 5 contained one major peak corresponding to mogroside compound 15. Fractions 1, 2 and 4 were further subjected to analytical-preparative separation described below, while fractions 3 and 5 were concentrated, dried and subjected to NMR and HPLCMS analyses.

Fractions 1, 2 and 4 from the preceding step were subjected to analytical-preparative HPLC, eluting with a mobile phase consisting of a mixture of acetonitrile and water with19:81 (vol/vol) ratio for fraction 1 and 25:75 (vol/vol) ratio for fractions 2 and 4. The column was Poroshell 120 SB-C18 ( 4.6 × 150 mm; 2.7µm), operated at 0.5 mL/min flowrate. The purified fractions containing individual peal<s were concentrated, dried and subjected to NMR and HPLCMS analyses.

### EXAMPLE 2 NMR analysis of the novel mogrosides

NMR samples were dissolved in methanol-d4 and analysed by 1D and 2D NMR methods. Noteworthy in the NMR analysis is the assignment of oxo- or hydroxyfunctional group at C11-for oxo- a typical 13C chemical shift around 190-220 ppm was seen, whereas for hydroxy- a typical 13C chemical shift of around 65-85 ppm was seen. Also noteworthy is the assignment of alpha-glycosidic linkages for some mogroside compounds where a coupling constant of approximately 3.5 Hz is seen (for beta-glycosidic linkages the coupling constant is usually approximately 7-9 Hz). All novel mogroside compounds claimed in this application showed good purity required for structural elucidation. The acquired data was used for structure elucidation.

### EXAMPLE 3

### HPLCMS analysis of the novel mogrosides

HPLCMS were performed on Shimadzu MS 2020 on Cortecs UPLC C18 1.6µm, 50×2.1 mm column, mobile phase A: 5mM ammonium formate +0,1% formic acid, B: methanokacetonitrile (1:1) +5mM ammonium phosphate buffer +0.1% formic acid, injection volume 0.1-5µl, detection ELSD (Sedex 85, pressure 4bar, nebulizer temperature 35°C), PDA 210-400 nm, gradient 5%B to 100%B in 4min followed by 2min 100% B, flowrate 1 ml/ min, scan 100-1600 amu, pos/neg switch. All mogroside compounds claimed in this application showed good purity required for HPLCMS analysis.

Based on NMR data acquired in Example 2 and the data acquired during HPLCMS analysis of novel mogrosides, the structures of mogroside compounds 1-15, including compounds (8), (9), (11), (12), (14) and (15) of the present invention, were proposed.

### EXAMPLE 4

### Novel mogrosides organoleptic characteristics

The organoleptic properties of the enriched composition comprising about 20% (w/w) mogrosides 1-15 (Example 1), the individual novel mogroside compounds, obtained from individual peak fractions of Example 1, were assessed in aqueous solutions isosweet to 5% sucrose. The assessment was conducted by 20 panelists in comparison with 5% isosweet aqueous solutions of commercial Luo Han Guo fruit extract of Example 1, highly purified (96.6% pure) Mogroside V sample (sourced from ChromaDex Inc., USA) and sucrose (5%). The results are summarized in Table 3 (compounds (8), (9), (11), (12), (14) and (15) being those of the present invention, other compounds being mentioned for the purposes of comparison only).

**Table 3**

| Novel Mogrosides (NM) Taste Properties | | | | | | |
|---|---|---|---|---|---|---|
| **Compounds** | **Sweetness potency (sucrose=1)** | **Sweetness Lingering*** | **Bitterness*** | **Delayed sweetness onset*** | **Licorice taste*** | **Overall taste profile vs. sugar** |
| Enriched composition (20% NM) | 320 | 3 | 2 | 3 | 2 | pleasant |
| NM 8 | 320 | 1 | 1 | 2 | 1 | sugar-like |
| NM 9 | 300 | 2 | 1 | 2 | 1 | sugar-like |
| NM 10 | 320 | 1 | 2 | 2 | 1 | sugar-like |
| NM 11 | 310 | 1 | 2 | 2 | 1 | sugar-like |
| NM 12 | 350 | I | 1 | 2 | 1 | sugar-like |
| NM 13 | 310 | 2 | 1 | 3 | 1 | sugar-like |
| NM 14 | 300 | 1 | 1 | 1 | I | sugar-like |
| NM 15 | 340 | 1 | 1 | 1 | 1 | sugar-like |
| Luo Han Guo extract | 300 | 5 | 5 | 5 | 5 | unpleasan t |
| Purified Mogroside V | 360 | 5 | 4 | 5 | 4 | tolerable |
| Sucrose | 1 | 1 | 1 | 1 | 1 | NA |

| | | | | | | |
|---|---|---|---|---|---|---|
| *For "Sweetness Lingering", "Bitterness", "Delayed sweetness onset", and "Licorice taste" characteristics the panelists score between 1 to 5, where the lower score represents more pleasant taste sensation by panelist | | | | | | |

The sensory evaluation results show all novel mogrosides possess significantly better taste profile compared to commercially available mogroside samples. The data for enriched composition (containing about 20% mogrosides 1-15) also shows that novel mogrosides can improve the overall taste characteristics of mogrosides mixtures comprising less pleasant mogrosides such as Mogroside V.

### EXAMPLE 5

### Consumable comprising novel mogrosides.

Carbonated beverage samples were prepared according to formula presented in Table 4.

**Table 4**

| Formula for carbonated beverages | |
|---|---|
| Ingredients | Quantity, % |
| Cola flavor | 0.340 |
| ortho-Phosphoric acid | 0.100 |
| Sodium citrate | 0.310 |
| Sodium benzoate | 0.018 |
| Citric acid | 0.018 |
| *Mogroside composition* | *0.050* |
| Carbonated water | to 100 |

The following samples were used as "mogroside composition" in the formula.

The enriched composition of Example 1 (comprising about 20% (w/w) mogrosides 1-15), the individual novel mogroside compounds, obtained from individual peak fractions of Example 1, commercial Luo Han Guo fruit extract of Example 1, highly purified (96.6% pure) Mogroside V sample (from ChromaDex Inc., USA). The sensory assessment of beverage samples was conducted by 20 panelists. The results are summarized in Table 5.

**Table 5**

| Sensory evaluation of carbonated beverage samples (compounds (8), (9), (11), (12), (14) and (15) being those of the present invention, other compounds being mentioned for the purposes of comparison only) | | | | | |
|---|---|---|---|---|---|
| **"Mogroside composition" used in formula** | **Sweetness Lingering*** | **Bitterness*** | **Delayed sweetness onset"** | **Licorice taste*** | **Overall taste** |
| Enriched composition (20% NM) | 2 | 2 | 2 | 2 | pleasant |
| NM 8 | 1 | 1 | 2 | 1 | sugar-like |
| NM9 | 2 | 1 | 1 | 1 | sugar-like |
| NM 10 | 1 | 2 | 2 | 1 | sugar-like |
| NM 11 | 2 | 1 | 1 | 1 | sugar-like |
| NM 12 | 1 | 1 | 2 | 1 | sugar-like |
| NM 13 | 1 | 2 | 2 | 1 | sugar-like |
| NM 14 | 1 | 1 | 1 | 2 | sugar-like |
| NM 15 | 2 | 1 | 1 | 1 | sugar-like |
| Luo Han Guo extract | 5 | 5 | 5 | 5 | unpleasant |
| Purified Mogroside V | 5 | 4 | 5 | 5 | tolerable |

| | | | | | |
|---|---|---|---|---|---|
| *For "Sweetness Lingering", "Bitterness", "Delayed sweetness onset", and "Licorice taste" characteristics the panelists score between 1 to 5, where the lower score represents more pleasant taste sensation by panelist | | | | | |

The results showed the beverages prepared using the composition comprising novel mogrosides possessed the best organoleptic characteristics.

### EXAMPLE 6

### Consumable comprising novel mogrosides.

Chocolate samples were papered according to formula in Table 6.

**Table 6**

| Formula for chocolate samples | |
|---|---|
| Ingredients | Quantity, % |
| Chocolate liquor | 30.0 |
| Cocoa butter | 11.5 |
| Milk powder | 14.0 |
| Sorbitol | 44.0 |
| Salt | 0.1 |
| *Mogroside composition* | *0.1* |
| Lecithin | 0.3 |

Chocolate liquor, cocoa butter, milk powder, sorbitol, salt, and the "mogroside composition" were kneaded sufficiently, and the mixture was then placed in a refiner to reduce its particle size for 24 hours. Thereafter, the content was transferred into a conche, the lecithin was added, and the composition was kneaded at 50°C for 48 hours. Then, the content was placed in a shaping apparatus, and solidified.

The following samples were used as "mogroside composition" in the formula of Table 6. Enriched composition comprising about 20% (w/w) mogrosides 1-15 (Example 1), the individual novel mogroside compounds, obtained from individual peak fractions of Example 1, commercial Luo Han Guo fruit extract of Example 1, highly purified (96.6% pure) Mogroside V sample sourced from ChromaDex Inc., (USA). The sensory assessment of chocolate samples was conducted by 20 panelists. The results are summarized in Table 7.

**Table 7**

| | | | | |
|---|---|---|---|---|
| Sensory evaluation of chocolate samples (compounds (8), (9), (11), (12), (14) and (15) being those of the present invention, other compounds being mentioned for the purposes of comparison only) | | | | |
| **"Mogroside composition" used in formula** | **Sweetness Lingering*** | **Bitterness*** | **Licorice taste*** | **Overall taste** |
| Enriched composition (20% NM) | 2 | 2 | 2 | pleasant |
| NM 8 | 1 | 1 | 1 | sugar-like |
| NM 9 | 2 | 2 | 1 | sugar-like |
| NM 10 | 1 | 2 | 2 | sugar-like |
| NM 11 | 1 | 1 | 1 | sugar-like |
| NM 12 | 2 | 1 | 2 | sugar-like |
| NM 13 | 1 | 2 | 1 | sugar-like |
| NM 14 | 1 | 1 | 2 | sugar-like |
| NM 15 | 2 | 2 | 1 | sugar-like |
| Luo Han Guo extract | 5 | 5 | 5 | unpleasant |
| Purified Mogroside V | 4 | 5 | 5 | tolerable |

| | | | | |
|---|---|---|---|---|
| *For "Sweetness Lingering", "Bitterness", and "Licorice taste" characteristics the panelists score between 1 to 5, where the lower score represents more pleasant taste sensation by panelist | | | | |

The results showed the chocolate samples prepared using the composition comprising novel mogrosides possessed the best organoleptic characteristics.

## Claims

1. Isolated or purified novel mogroside compounds having chemical structures represented in formulae 8, 9, 11, 12, 14 or 15:
| | |
|---|---|
| Formula 8 (Fig. 8) | 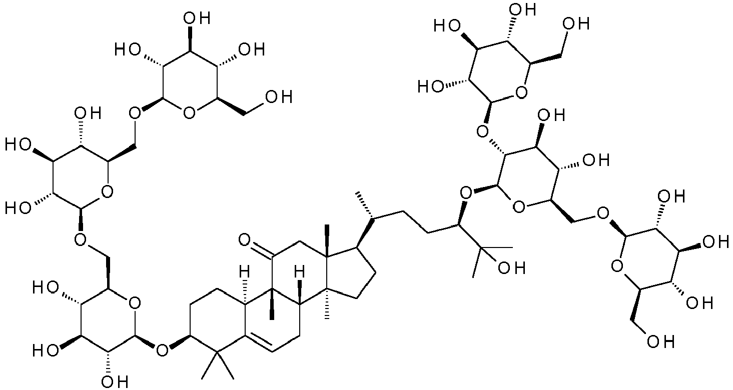 |
| Formula 9 (Fig. 9) | 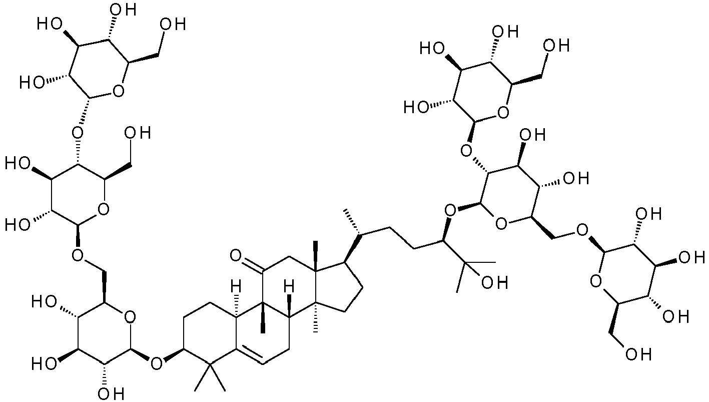 |
| Formula 11 (Fig. 11) | 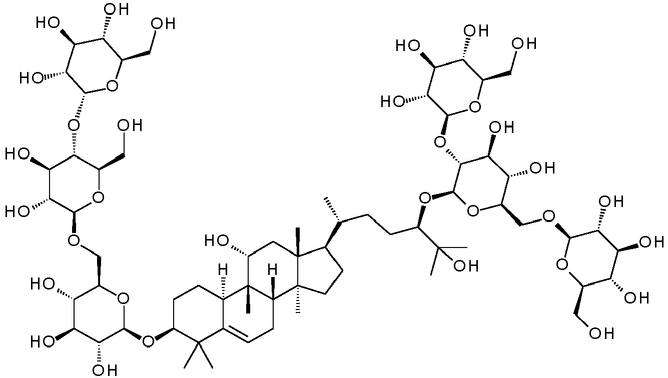 |
| Formula 12 (Fig. 12) | 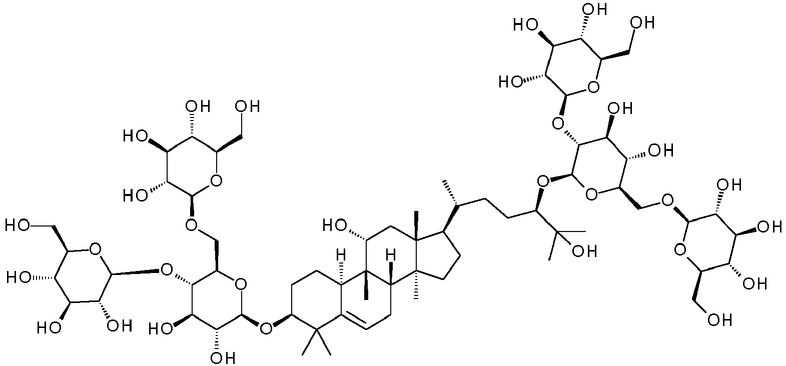 |
| Formula 14 (Fig. 14) | 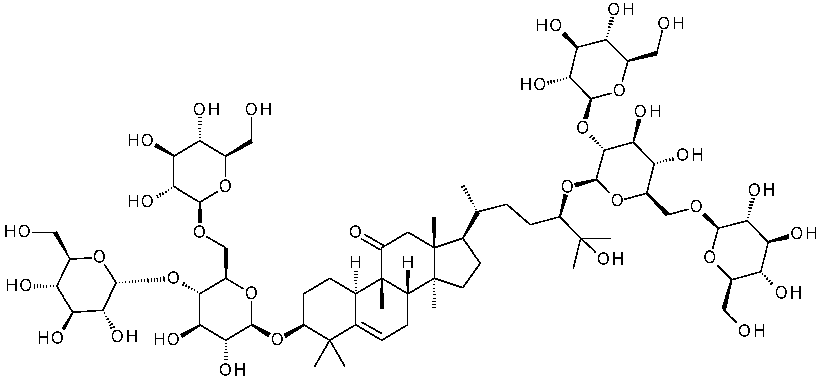 |
| Formula 15 (Fig. 15) | 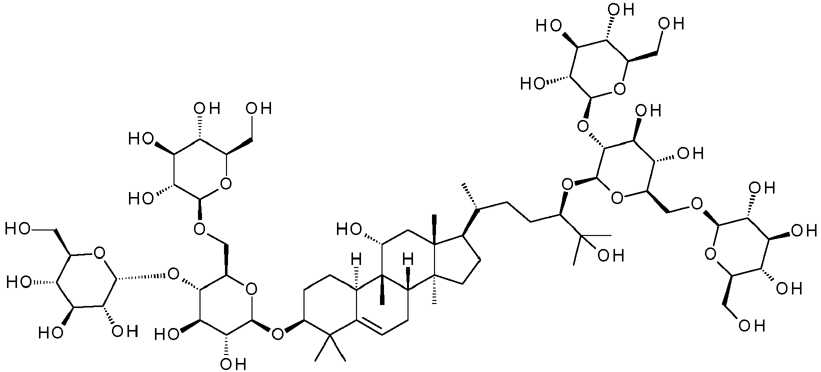 |

2. A composition comprising at least one compound of claim 1.

3. The composition of claim 2, further comprising
(i) mogrosides selected from the group consisting of Luo han guo extract, by-products of other mogrosides' isolation and purification processes, a commercially available Luo han guo extract, mogroside IA, mogroside IE, 11-oxomogroside IA, mogroside II A, mogroside II B, mogroside II E, 7-oxomogroside II E, mogroside III, 11-deoxymogroside III, mogroside IV, 11-oxomogroside IV A, mogroside V, 7-oxomogroside V, 11-oxomogroside V, isomogroside V, mogroside VI, mogrol, 11-oxomogrol, isomogroside, siamenoside, siamenoside I, neomogroside, synthetic mogrosides, other mogrosides occurring in *Siraitia grosvenorii* fruits and combinations thereof,
(ii) at least one additional sweetener, wherein the wherein the at least one additional sweetener is preferably selected from the group consisting of sucrose, glyceraldehyde, dihydroxyacetone, erythrose, threose, erythrulose, arabinose, lyxose, ribose, xylose, ribulose, xylulose, allose, altrose, allulose, galactose, glucose, gulose, idose, mannose, talose, fructose, psicose, sorbose, tagatose, mannoheptulose, sedoheltulose, octolose, fucose, rhamnose, arabinose, turanose, sialose, inulin, inulooligosaccharides, fructooligosaccharides, high fructose corn syrup (HFCS), maltodextrin, coupling sugar, honey, erythritol, xylitol, mannitol, sorbitol, inositol, stevia, rebaudioside A, rebaudioside B, rebaudioside C, rebaudioside D, rebaudioside E, rebaudioside F, rebaudioside G, rebaudioside H, rebaudioside I, rebaudioside J, rebaudioside K, rebaudioside L, rebaudioside M, rebaudioside N, rebaudioside O, dulcoside A, dulcoside B, rubusoside, steviolbioside, stevioside, other steviol glycosides occurring in *Stevia rebaudiana* plant, biosynthetic steviol glycosides, glycosylated steviol glycosides, glucosylated steviol glycosides (GSGs), mogroside IV, mogroside V, mogroside VI, Luo han guo, siamenoside, other mogrosides occurring in *Siraitia grosvenorii* fruits, monatin and its salts, curculin, glycyrrhizic acid and its salts, thaumatin, monellin, mabinlin, brazzein, hernandulcin, phyllodulcin, glycyphyllin, phloridzin, trilobatin, baiyunoside, osladin, polypodoside A, pterocaryoside A, pterocaryoside B, mukurozioside, phlomisoside I, periandrin I, abrusoside A, and cyclocarioside I, sugar alcohols, sucralose, potassium acesulfame, acesulfame acid and salts thereof, aspartame, alitame, saccharin and salts thereof, neohesperidin dihydrochalcone, naringin dihydrochalcone, cyclamate, cyclamic acid and salts thereof, neotame, advantame, and combinations thereof;
(iii) at least one additive selected from the group consisting of carbohydrates, polyols, amino acids and their corresponding salts, poly-amino acids and their corresponding salts, sugar acids and their corresponding salts, nucleotides, organic acids, inorganic acids, organic salts including organic acid salts and organic base salts, inorganic salts, bitter compounds, flavorants and flavoring ingredients, astringent compounds, proteins or protein hydrolysates, surfactants, emulsifiers, flavonoids, alcohols, polymers and combinations thereof; or
(iv) at least one functional ingredient selected from the group consisting of caffeine, saponins, antioxidants, dietary fiber sources, fatty acids, vitamins, glucosamine, minerals, preservatives, hydration agents, probiotics, prebiotics, weight management agents, osteoporosis management agents, phytoestrogens, long chain primary aliphatic saturated alcohols, phytosterols and combinations thereof.

4. A consumable comprising at least one compound of claim 1, wherein the consumable is preferably a beverage or food product.

5. The composition of claim 2, further comprising at least one flavor ingredient, wherein the concentration of at least one compound of claim 1 is below the threshold flavor recognition concentration of the compound.

6. A method for enhancing the flavor of a consumable comprising (i) providing a consumable comprising at least one flavor ingredient and (ii) adding at least one compound of claim 1 to the consumable to provide a consumable with enhanced flavor, wherein at least one compound of claim 1 is present in the consumable with enhanced flavor at a concentration below the threshold flavor recognition concentration of the compound.

7. The method of claim 6, wherein the consumable is a beverage.

8. The composition of claim 2, further comprising at least one sweetener, wherein at least one compound of claim 1 is present in the composition in a concentration below the threshold sweetness recognition concentration of the compound.

9. The composition of claim 8, wherein the sweetener is selected from the group consisting of sucrose, glyceraldehyde, dihydroxyacetone, erythrose, threose, erythrulose, arabinose, lyxose, ribose, xylose, ribulose, xylulose, allose, altrose, allulose, galactose, glucose, gulose, idose, mannose, talose, fructose, psicose, sorbose, tagatose, mannoheptulose, sedoheltulose, octolose, fucose, rhamnose, arabinose, turanose, sialose, inulin, inulooligosaccharides, fructooligosaccharides, HFCS, maltodextrin, coupling sugar, honey, stevia, rebaudioside A, rebaudioside B, rebaudioside C, rebaudioside D, rebaudioside E, rebaudioside F, rebaudioside G, rebaudioside H, rebaudioside I, rebaudioside J, rebaudioside K, rebaudioside L, rebaudioside M, rebaudioside N, rebaudioside O, dulcoside A, dulcoside B, rubusoside, steviolbioside, stevioside, other steviol glycosides occurring in *Stevia rebaudiana* plant, biosynthetic steviol glycosides, glycosylated steviol glycosides, glucosylated steviol glycosides (GSGs), mogroside IV, mogroside V, mogroside VI, Luo han guo, siamenoside, other mogrosides occurring in *Siraitia grosvenorii* fruits, monatin and its salts, curculin, glycyrrhizic acid and its salts, thaumatin, monellin, mabinlin, brazzein, hernandulcin, phyllodulcin, glycyphyllin, phloridzin, trilobatin, baiyunoside, osladin, polypodoside A, pterocaryoside A, pterocaryoside B, mukurozioside, phlomisoside I, periandrin I, abrusoside A, and cyclocarioside I, sugar alcohols, sucralose, potassium acesulfame, acesulfame acid and salts thereof, aspartame, alitame, saccharin and salts thereof, neohesperidin dihydrochalcone, cyclamate, cyclamic acid and salts thereof, neotame, advantame, and combinations thereof.

10. A method for enhancing the sweetness of a consumable comprising (i) providing a consumable comprising at least one sweetener and (ii) adding at least one compound of claim 1 to the consumable to provide a consumable with enhanced sweetness, wherein at least one compound of claim 1 is present in the consumable with enhanced sweetness in a concentration below the threshold sweetness recognition concentration of the compound.

11. The method of claim 10, wherein the consumable is a beverage.

12. A method for purifying at least one compound of claim 1 comprising:
(a) passing a solution comprising at least one compound of claim 1 through a chromatographic system; and
(b) collecting fractions comprising at least one compound of claim 1.

13. The method of claim 12, further comprising removal of solvents from the collected fractions.

14. The method of claim 12, wherein the solution comprises at least one solvent selected from the group consisting of water, ethanol, acetonitrile, methanol, 2-propanol, ethylacetate, dimethylformamide, dimethylsulfide, pyridine, triethylamine, formic acid, trifluoroacetic acid, acetic acid, an aqueous solution containing ammonium acetate, heptafluorobutyric acid, and any combination thereof.

15. The method of claim 12, wherein the chromatographic system is preparative HPLC operating under a gradient of mobile phase.

## Patentansprüche

1. Isolierte oder gereinigte neuartige Mogrosidverbindungen, die chemische Strukturen aufweisen, die in den Formeln 8, 9, 11, 12, 14 oder 15 dargestellt sind:
| | |
|---|---|
| Formel 8 (Fig. 8) | 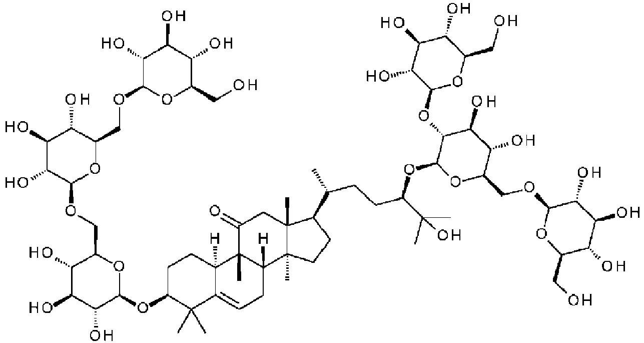 |
| Formel 9 (Fig. 9) | 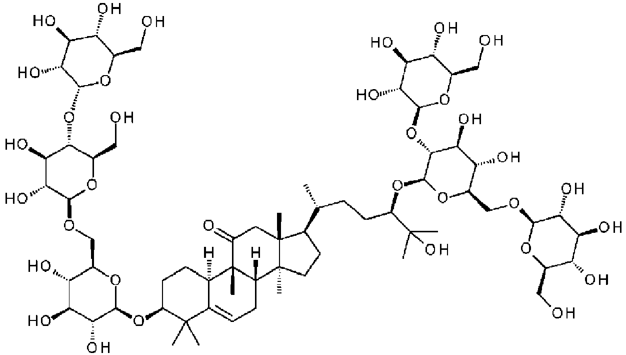 |
| Formel 11 (Fig. 11) | 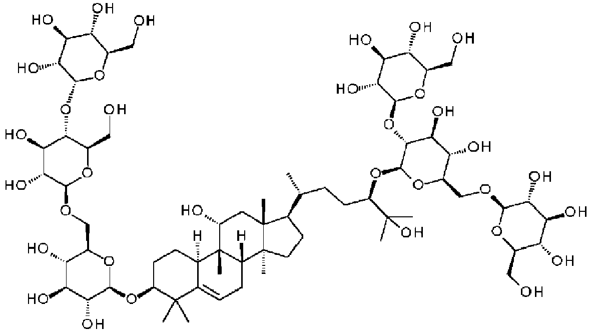 |
| Formel 12 (Fig. 12) | 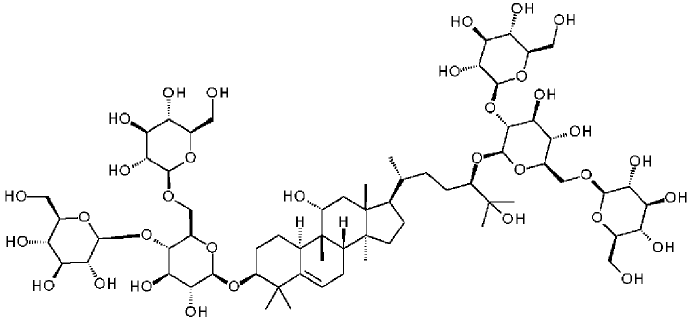 |
| Formel 14 (Fig. 14) | 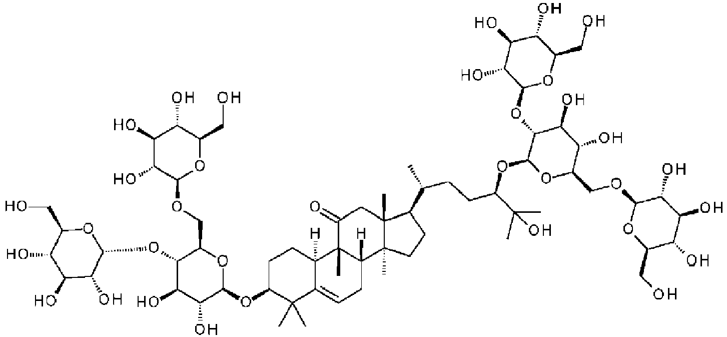 |
| Formel 15 (Fig. 15) | 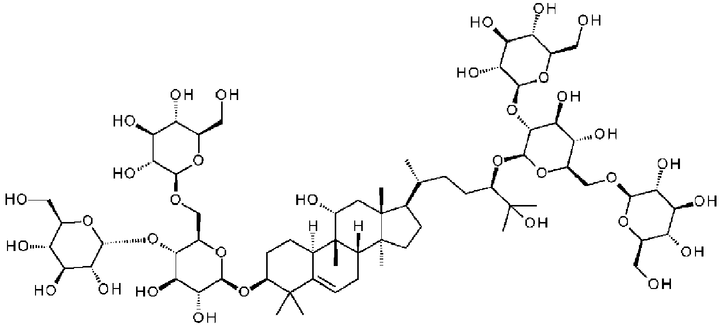 |

2. Zusammensetzung, umfassend mindestens eine Verbindung nach Anspruch 1.

3. Zusammensetzung nach Anspruch 2, ferner umfassend
(i) Mogroside, die aus der Gruppe ausgewählt sind, bestehend aus Luo-Han-Guo-Extrakt, Nebenprodukten anderer Trenn- und Reinigungsverfahren von Mogrosiden, einem handelsüblichen Luo-Han-Guo-Extrakt, Mogrosid IA, Mogrosid IE, 11-Oxomogrosid IA, Mogrosid II A, Mogrosid II B, Mogrosid II E, 7-Oxomogrosid II E, Mogrosid III, 11-Deoxymogrosid III, Mogrosid IV, 11-Oxomogrosid IV A, Mogrosid V, 7-Oxomogrosid V, 11-Oxomogrosid V, Isomogrosid V, Mogrosid VI, Mogrol, 11-Oxomogrol, Isomogrosid, Siamenosid, Siamenosid I, Neomogrosid, synthetischen Mogrosiden, anderen Mogrosiden, die in *Siraitia* grosvenorii-Früchten und Kombinationen davon auftreten,
(ii) mindestens einen zusätzlichen Süßstoff, wobei der wobei der mindestens eine zusätzliche Süßstoff vorzugsweise aus der Gruppe ausgewählt ist, bestehend aus Sucrose, Glyceraldehyd, Dihydroxyaceton, Erythrose, Threose, Erythrulose, Arabinose, Lyxose, Ribose, Xylose, Ribulose, Xylulose, Allose, Altrose, Allulose, Galactose, Glucose, Gulose, Idose, Mannose, Talose, Fructose, Psicose, Sorbose, Tagatose, Mannoheptulose, Sedoheltulose, Octolose, Fucose, Rhamnose, Arabinose, Turanose, Sialose, Inulin, Inulooligosacchariden, Fructooligosacchariden, Isoglucosesirup (HFCS), Maltodextrin, Kupplungszucker, Honig, Erythrit, Xylit, Mannit, Sorbit, Inosit, Stevia, Rebaudiosid A, Rebaudiosid B, Rebaudiosid C, Rebaudiosid D, Rebaudiosid E, Rebaudiosid F, Rebaudioside G, Rebaudiosid H, Rebaudiosid I, Rebaudiosid J, Rebaudiosid K, Rebaudiosid L, Rebaudiosid M, Rebaudiosid N, Rebaudiosid O, Dulcosid A, Dulcosid B, Rubusosid, Steviolbiosid, Steviosid, anderen Steviolglycosiden, die in einer *Stevia* rebaudiana-Pflanze auftreten, biosynthetischen Steviolglycosiden, glycosylierten Steviolglycosiden, glucosylierten Steviolglycosiden (GSGs), Mogrosid IV, Mogrosid V, Mogrosid VI; Luo Han Guo, Siamenosid, anderen Mogrosiden, die in *Siraitia* grosvenorii-Früchten auftreten, Monatin und seinen Salzen, Curculin, Glycyrrhizinsäure und ihren Salzen, Thaumatin, Monellin, Mabinlin, Brazzein, Hernandulcin, Phyllodulcin, Glycyphyllin, Phloridzin, Trilobatin, Baiyunosid, Osladin, Polypodosid A, Pterocaryosid A, Pterocaryosid B, Mukuroziosid, Phlomisosid I, Periandrin I, Abrusosid A und Cyclocariosid I, Zuckeralkoholen, Sucralose, Kaliumacesulfam, Acesulfamsäure und Salzen davon, Aspartam, Alitam, Saccharin und Salzen davon, Neohesperidindihydrochalcon, Naringindihydrochalcon, Cyclamat, Cyclaminsäure und Salzen davon, Neotam, Advantam und Kombinationen davon;
(iii) mindestens ein Zusatzstoff, der aus der Gruppe ausgewählt ist, bestehend aus Kohlenhydraten, Polyolen, Aminosäuren und ihren entsprechenden Salzen, Polyaminosäuren und ihren entsprechenden Salzen, Zuckersäuren und ihren entsprechenden Salzen, Nucleotiden, organischen Säuren, anorganischen Säuren, organischen Salzen einschließlich organischer Säuresalze und organischen Basissalzen, anorganischen Salzen, Bitterstoffen, Geschmacksstoffen und geschmacksgebenden Inhaltsstoffen, adstringierenden Verbindungen, Proteinen oder Proteinhydrolysaten, Tensiden, Emulgatoren, Flavonoiden, Alkoholen, Polymeren und Kombinationen davon; oder
(iv) mindestens einen funktionellen Inhaltsstoff, der aus der Gruppe ausgewählt ist, bestehend aus Coffein, Saponin, Antioxidantien, Ballastfaserquellen, Fettsäuren, Vitaminen, Glucosamin, Mineralien, Konservierungsmitteln, Hydratationsmitteln, Probiotika, Präbiotika, Gewichtskontrollmitteln, Osteoporosekontrollmitteln, Phytostrogenen, langkettigen primären aliphatischen gesättigten Alkoholen, Phytosterolen und Kombinationen davon.

4. Verbrauchsmaterial, umfassend mindestens eine Verbindung nach Anspruch 1, wobei das Verbrauchsmaterial vorzugsweise ein Getränke- oder Lebensmittelprodukt ist.

5. Zusammensetzung nach Anspruch 2, ferner umfassend mindestens einen Geschmacksinhaltsstoff, wobei die Konzentration von mindestens einer Verbindung nach Anspruch 1 unter der Schwellenwerterkennungskonzentration der Verbindung liegt.

6. Verfahren zum Verbessern des Geschmacks eines Verbrauchsmaterials, umfassend (i) ein Bereitstellen eines Verbrauchsmaterials, umfassend mindestens einen Geschmacksinhaltsstoff, und (ii) ein Hinzufügen mindestens einer Verbindung nach Anspruch 1 zu dem Verbrauchsmaterial, um ein Verbrauchsmaterial mit verbessertem Geschmack bereitzustellen, wobei mindestens eine Verbindung nach Anspruch 1 in dem Verbrauchsmaterial mit verbessertem Geschmack in einer Konzentration unterhalb der Schwellenwertgeschmackserkennungskonzentration der Verbindung vorhanden ist.

7. Verfahren nach Anspruch 6, wobei das Verbrauchsmittel ein Getränk ist.

8. Zusammensetzung nach Anspruch 2, ferner umfassend mindestens einen Süßstoff, wobei mindestens eine Verbindung nach Anspruch 1 in der Zusammensetzung in einer Konzentration unterhalb der Schwellenwertsüßeerkennungskonzentration der Verbindung vorhanden ist.

9. Zusammensetzung nach Anspruch 8, wobei der Süßstoff aus der Gruppe ausgewählt ist, bestehend aus Sucrose, Glyceraldehyd, Dihydroxyaceton, Erythrose, Threose, Erythrulose, Arabinose, Lyxose, Ribose, Xylose, Ribulose, Xylulose, Allose, Altrose, Allulose, Galactose, Glucose, Gulose, Idose, Mannose, Talose, Fructose, Psicose, Sorbose, Tagatose, Mannoheptulose, Sedoheltulose, Octolose, Fucose, Rhamnose, Arabinose, Turanose, Sialose, Inulin, Inulooligosacchariden, Fructooligosacchariden, HFCS, Maltodextrin, Kupplungszucker, Honig, Stevia, Rebaudiosid A, Rebaudiosid B, Rebaudiosid C, Rebaudiosid D, Rebaudiosid E, Rebaudiosid F, Rebaudioside G, Rebaudiosid H, Rebaudiosid I, Rebaudiosid J, Rebaudiosid K, Rebaudiosid L, Rebaudiosid M, Rebaudiosid N, Rebaudiosid O, Dulcosid A, Dulcosid B, Rubusosid, Steviolbiosid, Steviosid, anderen Steviolglycosiden, die in einer *Stevia rebaudiana*-Pflanze auftreten, biosynthetischen Steviolglycosiden, glycosylierten Steviolglycosiden, glucosylierten Steviolglycosiden (GSGs), Mogrosid IV, Mogrosid V, Mogrosid VI; Luo Han Guo, Siamenosid, anderen Mogrosiden, die in *Siraitia* grosvenorii-Früchten auftreten, Monatin und seinen Salzen, Curculin, Glycyrrhizinsäure und ihren Salzen, Thaumatin, Monellin, Mabinlin, Brazzein, Hernandulcin, Phyllodulcin, Glycyphyllin, Phloridzin, Trilobatin, Baiyunosid, Osladin, Polypodosid A, Pterocaryosid A, Pterocaryosid B, Mukuroziosid, Phlomisosid I, Periandrin I, Abrusosid A und Cyclocariosid I, Zuckeralkoholen, Sucralose, Kaliumacesulfam, Acesulfamsäure und Salzen davon, Aspartam, Alitam, Saccharin und Salzen davon, Neohesperidindihydrochalcon, Cyclamat, Cyclaminsäure und Salzen davon, Neotam, Advantam und Kombinationen davon.

10. Verfahren zum Verbessern der Süße eines Verbrauchsmaterials, umfassend (i) das Bereitstellen eines Verbrauchsmaterials, umfassend mindestens einen Süßstoff, und (ii) das Hinzufügen mindestens einer Verbindung nach Anspruch 1 zu dem Verbrauchsmaterial, um ein Verbrauchsmaterial mit verbesserter Süße bereitzustellen, wobei mindestens eine Verbindung nach Anspruch 1 in dem Verbrauchsmaterial mit verbesserter Süße in einer Konzentration unter der Schwellenwertsüßeerkennungskonzentration der Verbindung vorhanden ist.

11. Verfahren nach Anspruch 10, wobei das Verbrauchsmittel ein Getränk ist.

12. Verfahren zum Reinigen mindestens einer Verbindung nach Anspruch 1, umfassend:
(a) Leiten einer Lösung, umfassend mindestens eine Verbindung nach Anspruch 1, über ein chromatografisches System; und
(b) Sammeln von Fraktionen, umfassend mindestens eine Verbindung nach Anspruch 1.

13. Verfahren nach Anspruch 12, ferner umfassend eine Entfernung von Lösungsmitteln aus den gesammelten Fraktionen.

14. Verfahren nach Anspruch 12, wobei die Lösung mindestens ein Lösungsmittel umfasst, das aus der Gruppe ausgewählt ist, bestehend aus Wasser, Ethanol, Acetonitril, Methanol, 2-Propanol, Ethylacetat, Dimethylformamid, Dimethylsulfid, Pyridin, Triethylamin, Ameisensäure, Trifluoressigsäure, Essigsäure, einer wässrigen Lösung, die Ammoniumacetat, Heptafluorbuttersäure und eine beliebige Kombination davon enthält.

15. Verfahren nach Anspruch 12, wobei das chromatographische System eine präparative HPLC ist, die unter einem Gradienten einer mobilen Phase arbeitet.

## Revendications

1. Nouveaux composés de mogroside isolés ou purifiés ayant les structures chimiques représentées dans les formules 8, 9, 11, 12, 14 ou 15 :
| | |
|---|---|
| Formule 8 (Fig. 8) | 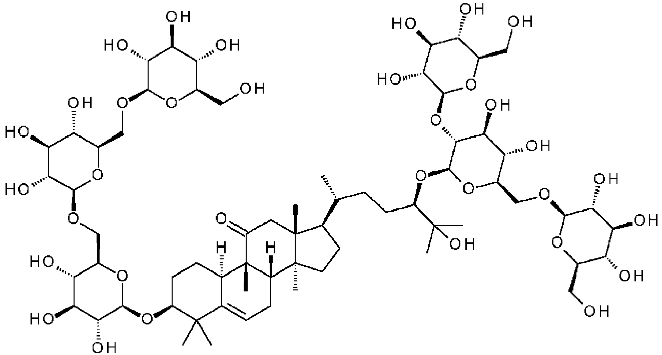 |
| Formule 9 (Fig. 9) | 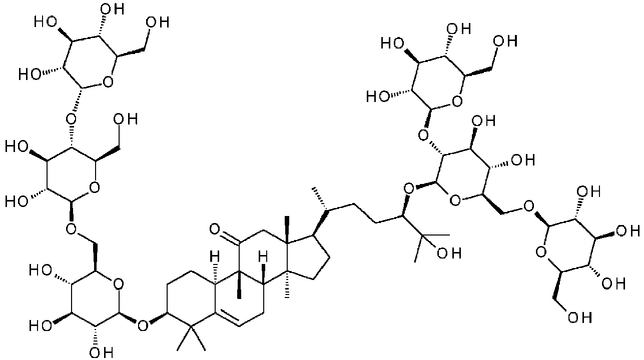 |
| Formule 11 (Fig. 11) | 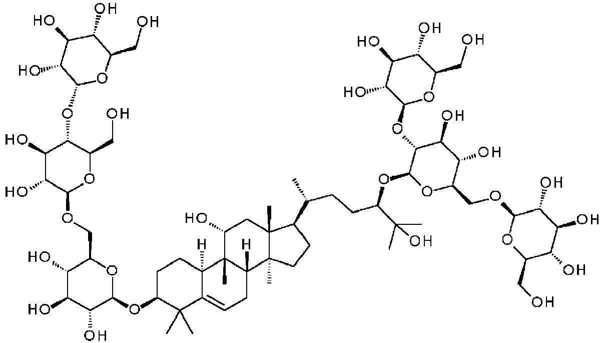 |
| Formule 12 (Fig. 12) | 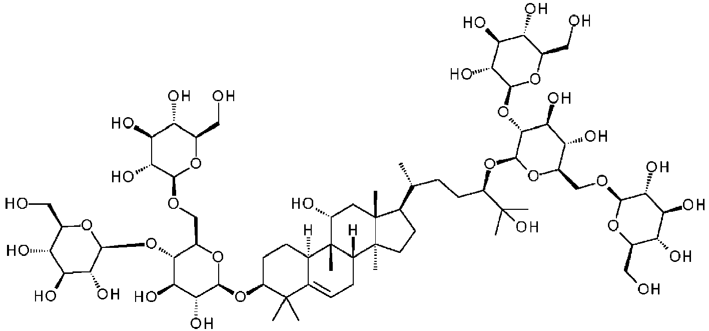 |
| Formule 14 (Fig. 14) | 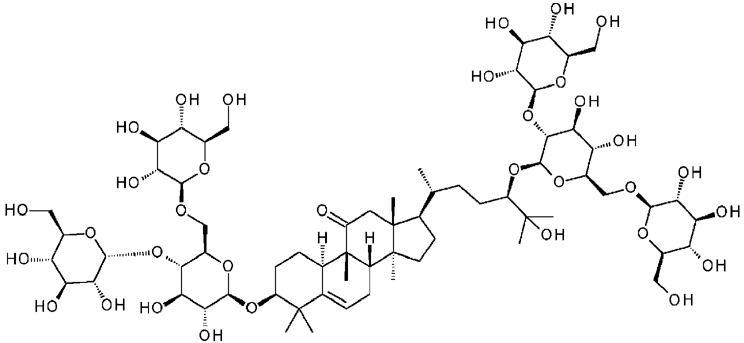 |
| Formule 15 (Fig. 15) | 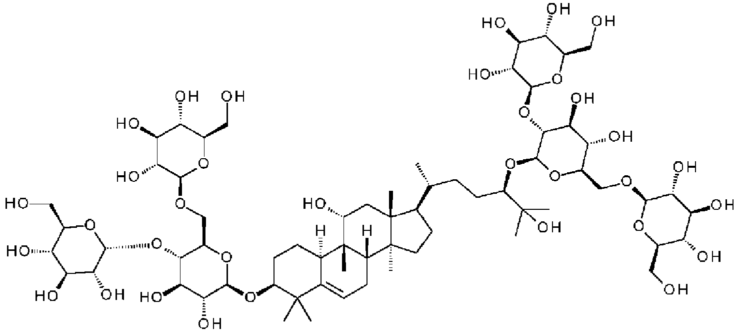 |

2. Composition comprenant au moins un composé selon la revendication 1.

3. Composition selon la revendication 2, comprenant en outre
(i) des mogrosides choisis dans le groupe constitué d'extrait de Luo han guo, sous-produits d'autres processus d'isolation et de purification de mogrosides, extrait de Luo han guo disponible dans le commerce, mogroside IA, mogroside IE, 11-oxomogroside IA, mogroside II A, mogroside II B, mogroside II E, 7-oxomogroside II E, mogroside III, 11-désoxymogroside III, mogroside IV, 11-oxomogroside IV A, mogroside V, 7-oxomogroside V, 11-oxomogroside V, isomogroside V, mogroside VI, mogrol, 11-oxomogrol, isomogroside, siaménoside, siaménoside I, néomogroside, mogrosides synthétiques, autres mogrosides présents dans des fruits de *Siraitia grosvenorii* et combinaisons de ceux-ci,
(ii) au moins un édulcorant supplémentaire, dans laquelle le dans laquelle l'au moins un édulcorant supplémentaire est de préférence choisi dans le groupe constitué de saccharose, glycéraldéhyde, dihydroxyacétone, érythrose, thréose, érythrulose, arabinose, lyxose, ribose, xylose, ribulose, xylulose, allose, altrose, allulose, galactose, glucose, gulose, idose, mannose, talose, fructose, psicose, sorbose, tagatose, mannoheptulose, sédoheltulose, octolose, fucose, rhamnose, arabinose, turanose, sialose, inuline, inulooligosaccharides, fructooligosaccharides, sirop de maïs à haute teneur en fructose (HFCS), maltodextrine, coupling sugar, miel, érythritol, xylitol, mannitol, sorbitol, inositol, stévia, rébaudioside A, rébaudioside B, rébaudioside C, rébaudioside D, rébaudioside E, rébaudioside F, rébaudioside G, rébaudioside H, rébaudioside I, rébaudioside J, rébaudioside K, rébaudioside L, rébaudioside M, rébaudioside N, rébaudioside O, dulcoside A, dulcoside B, rubusoside, stéviolbioside, stévioside, autres glycosides de stéviol présents dans une plante de *Stevia rebaudiana,* glycosides de stéviol biosynthétiques, glycosides de stéviol glycosylés, glycosides de stéviol glucosylés (GSG), mogroside IV, mogroside V, mogroside VI, Luo han guo, siaménoside, autres mogrosides présents dans des fruits de *Siraitia grosvenorii,* monatine et ses sels, curculine, acide glycyrrhizique et ses sels, thaumatine, monelline, mabinline, brazzéine, hernandulcine, phyllodulcine, glycyphylline, phloridzine, trilobatine, baiyunoside, osladine, polypodoside A, ptérocaryoside A, ptérocaryoside B, mukurozioside, phlomisoside I, périandrine I, abrusoside A et cyclocarioside I, alcools de sucre, sucralose, acésulfame de potassium, acide acésulfame et sels de celui-ci, aspartame, alitame, saccharine et sels de celle-ci, néohespéridine dihydrochalcone, naringine dihydrochalcone, cyclamate, acide cyclamique et sels de celui-ci, néotame, advantame, et combinaisons de ceux-ci ;
(iii) au moins un additif choisi dans le groupe constitué de glucides, polyols, acides aminés et leurs sels correspondants, acides polyaminés et leurs sels correspondants, acides de sucre et leurs sels correspondants, nucléotides, acides organiques, acides inorganiques, sels organiques, y compris sels d'acides organiques et sels de bases organiques, sels inorganiques, composés amers, agents de saveur et ingrédients de saveur, composés astringents, protéines ou hydrolysats de protéines, tensioactifs, émulsifiants, flavonoïdes, alcools, polymères et combinaisons de ceux-ci ; ou
(iv) au moins un ingrédient fonctionnel choisi dans le groupe constitué de caféine, saponines, antioxydants, sources de fibres alimentaires, acides gras, vitamines, glucosamine, minéraux, conservateurs, agents d'hydratation, probiotiques, prébiotiques, agents de gestion du poids, agents de gestion de l'ostéoporose, phytoestrogènes, alcools saturés aliphatiques primaires à longue chaîne, phytostérols et combinaisons de ceux-ci.

4. Produit consommable comprenant au moins un composé selon la revendication 1, dans lequel le produit consommable est de préférence un produit de boisson ou alimentaire.

5. Composition selon la revendication 2, comprenant en outre au moins un ingrédient de saveur, dans laquelle la concentration d'au moins un composé selon la revendication 1 est en dessous de la concentration seuil de reconnaissance de saveur du composé.

6. Procédé permettant d'améliorer la saveur d'un produit consommable comprenant (i) la fourniture d'un produit consommable comprenant au moins un ingrédient de saveur et (ii) l'ajout d'au moins un composé selon la revendication 1 au produit consommable pour fournir un produit consommable à saveur améliorée, dans lequel au moins un composé selon la revendication 1 est présent dans le produit consommable à saveur améliorée à une concentration en dessous de la concentration seuil de reconnaissance de saveur du composé.

7. Procédé selon la revendication 6, dans lequel le produit consommable est une boisson.

8. Composition selon la revendication 2, comprenant en outre au moins un édulcorant, dans lequel au moins un composé selon la revendication 1 est présent dans la composition à une concentration en dessous de la concentration seuil de reconnaissance de saveur du composé.

9. Composition selon la revendication 8, dans laquelle l'édulcorant est de préférence choisi dans le groupe constitué de saccharose, glycéraldéhyde, dihydroxyacétone, érythrose, thréose, érythrulose, arabinose, lyxose, ribose, xylose, ribulose, xylulose, allose, altrose, allulose, galactose, glucose, gulose, idose, mannose, talose, fructose, psicose, sorbose, tagatose, mannoheptulose, sédoheltulose, octolose, fucose, rhamnose, arabinose, turanose, sialose, inuline, inulooligosaccharides, fructooligosaccharides, HFCS, maltodextrine, coupling sugar, miel, stévia, rébaudioside A, rébaudioside B, rébaudioside C, rébaudioside D, rébaudioside E, rébaudioside F, rébaudioside G, rébaudioside H, rébaudioside I, rébaudioside J, rébaudioside K, rébaudioside L, rébaudioside M, rébaudioside N, rébaudioside O, dulcoside A, dulcoside B, rubusoside, stéviolbioside, stévioside, autres glycosides de stéviol présents dans une plante de *Stevia rebaudiana,* glycosides de stéviol biosynthétiques, glycosides de stéviol glycosylés, glycosides de stéviol glucosylés (GSG), mogroside IV, mogroside V, mogroside VI, Luo han guo, siaménoside, autres mogrosides présents dans des fruits de *Siraitia grosvenorii,* monatine et ses sels, curculine, acide glycyrrhizique et ses sels, thaumatine, monelline, mabinline, brazzéine, hernandulcine, phyllodulcine, glycyphylline, phloridzine, trilobatine, baiyunoside, osladine, polypodoside A, ptérocaryoside A, ptérocaryoside B, mukurozioside, phlomisoside I, périandrine I, abrusoside A et cyclocarioside I, alcools de sucre, sucralose, acésulfame de potassium, acide acésulfame et sels de celui-ci, aspartame, alitame, saccharine et sels de celle-ci, néohespéridine dihydrochalcone, cyclamate, acide cyclamique et sels de celui-ci, néotame, advantame, et combinaisons de ceux-ci.

10. Procédé permettant d'améliorer la sucrosité d'un produit consommable comprenant (i) la fourniture d'un produit consommable comprenant au moins un édulcorant et (ii) l'ajout d'au moins un composé selon la revendication 1 au produit consommable pour fournir un produit consommable à sucrosité améliorée, dans lequel au moins un composé selon la revendication 1 est présent dans le produit consommable à sucrosité améliorée à une concentration en dessous de la concentration seuil de reconnaissance de saveur du composé.

11. Procédé selon la revendication 10, dans lequel le produit consommable est une boisson.

12. Procédé permettant de purifier au moins un composé selon la revendication 1 comprenant :
(a) le passage d'une solution comprenant au moins un composé selon la revendication 1 à travers un système chromatographique ; et
(b) la collecte de fractions comprenant au moins un composé selon la revendication 1.

13. Procédé selon la revendication 12, comprenant en outre l'élimination de solvants des fractions collectées.

14. Procédé selon la revendication 12, dans lequel la solution comprend au moins un solvant choisi dans le groupe constitué d'eau, éthanol, acétonitrile, méthanol, 2-propanol, acétate d'éthyle, diméthylformamide, sulfure de diméthyle, pyridine, triéthylamine, acide formique, acide trifluoroacétique, acide acétique, une solution aqueuse contenant de l'acétate d'ammonium, acide heptafluorobutyrique, et n'importe quelle combinaison de ceux-ci.

15. Procédé selon la revendication 12, dans lequel le système chromatographique est une HPLC préparative fonctionnant sous un gradient de phase mobile.
